# EUROPEAN PATENT SPECIFICATION

(11) **EP 2 120 924 B1**
(45) Date of publication and mention of the grant of the patent: **09.03.2016**
(21) Application number: 08717610.3
(22) Date of filing: 11.03.2008
(51) Int. Cl.: A61K 36/00, A61K 31/7048, A61K 36/48, A61K 31/352

(54) **AN ANTI-DIABETIC EXTRACT OF HONEYBUSH**
ANTI-DIABETISCHER HONEYBUSH EXTRAKT
EXTRAIT ANTI-DIABÉTIQUE DE HONEYBUSH

(30) Priority: 12.03.2007 US 894256 P
(43) Date of publication of application: 25.11.2009
(73) Proprietor: Zadec APS, 2900 Hellerup (DK)
(72) Inventor: LARSEN, Peter Mose, DK-5260 Odense S (DK); FEY, Stephen J, DK-5491 Blommenslyst (DK); LOUW, Johan, 7505 Tygerberg (ZA); JOUBERT, Lizette, 0001 Pretoria (ZA)
(74) Representative: Orsnes, Henrik Egede
(86) International application number: PCT/EP2008/052863
(87) International publication number: WO 2008/110552

(56) References cited:
- MCKAY DIANE L ET AL: "A review of the bioactivity of South African herbal teas: Rooibos (Aspalathus linearis) and honeybush (Cyclopia intermedia)" PHYTOTHERAPY RESEARCH, JOHN WILEY & SONS LTD. CHICHESTER, GB, vol. 21, no. 1, 1 January 2007 (2007-01-01), pages 1-16, XP002500806 ISSN: 0951-418X
- DE NYSSCHEN A M ET AL: "The major phenolic compounds in the leaves of Cyclopia species (Honeybush tea)" BIOCHEMICAL SYSTEMATICS AND ECOLOGY, PERGAMON PRESS, GB, vol. 24, 1 January 1996 (1996-01-01), pages 243-246, XP002505919 ISSN: 0305-1978
- KAMARA B I ET AL: "Phenolic metabolites from Honeybush Tea (Cyclopia subternata" JOURNAL OF AGRICULTURAL AND FOOD CHEMISTRY, AMERICAN CHEMICAL SOCIETY. WASHINGTON.; US, vol. 52, no. 17, 28 July 2004 (2004-07-28), pages 5391-5395, XP002368627 ISSN: 0021-8561
- MARNEWICK J ET AL: "Inhibition of tumour promotion in mouse skin by extracts of rooibos (Aspalathus linearis) and honeybush (Cyclopia intermedia), unique South African herbal teas" CANCER LETTERS, NEW YORK, NY, US, vol. 224, no. 2, 28 June 2005 (2005-06-28), pages 193-202, XP004899142 ISSN: 0304-3835
- VAN DER MERWE J D ET AL: "A comparative study on the antimutagenic properties of aqueous extracts of Aspalathus linearis (rooibos), different Cyclopia spp. (honeybush) and Camellia sinensis teas" MUTATION RESEARCH. GENETIC TOXICOLOGY AND ENVIRONMENTALMUTAGENESIS, ELSEVIER, AMSTERDAM, NL, vol. 611, no. 1-2, 10 December 2006 (2006-12-10), pages 42-53, XP025175685 ISSN: 1383-5718 [retrieved on 2006-12-10]
- JOUBERT ELIZABETH ET AL: "Evaluation of spectrophotometric methods for screening of green rooibos (Aspalathus linearis) and green honeybush (Cyclopia genistoides) extracts for high levels of Bio-active compounds." PHYTOCHEMICAL ANALYSIS : PCA MAR 2008, vol. 19, no. 2, March 2008 (2008-03), pages 169-178, XP002514565 ISSN: 1099-1565
- MIURA T ET AL: "The suppressive effect mangiferin with exercise on blood lipids in type 2 diabetes" BIOLOGICAL AND PHARMACEUTICAL BULLETIN 2001 JP, vol. 24, no. 9, 2001, pages 1091-1092, XP002531643 ISSN: 0918-6158
- MIURA T ET AL: "Antidiabetic activity of a xanthone compound, mangiferin" PHYTOMEDICINE, GUSTAV FISCHER VERLAG, STUTTGART, vol. 8, no. 2, 1 January 2001 (2001-01-01), pages 85-87, XP004957205 ISSN: 0944-7113
- MIURA TOSHIHIRO ET AL: "Antidiabetic activity of the rhizoma of Anemarrhena asphodeloides and active components, mangiferin and its glucoside" BIOLOGICAL AND PHARMACEUTICAL BULLETIN, vol. 24, no. 9, September 2001 (2001-09), pages 1009-1011, XP002531644 ISSN: 0918-6158
- JOUBERT ELIZABETH ET AL: "Use of NIRS for quantification of mangiferin and hesperidin contents of dried green honeybush (Cyclopia genistoides) plant material" JOURNAL OF AGRICULTURAL AND FOOD CHEMISTRY, vol. 54, no. 15, July 2006 (2006-07), pages 5279-5283, XP002531645 ISSN: 0021-8561
- Hiroyuki Ichiki ET AL: "New Antidiabetic Compounds, Magniferin and Its Glucoside", Biol. Pharm. Bull., vol. 21, no. 12, 1 December 1998 (1998-12-01), pages 1389-1390, XP055143756,

## Description

### FIELD OF THE INVENTION

The present application concerns the field of natural products and more specifically plant extracts and compounds useful for the treatment of diabetes.

### BACKGROUND OF THE INVENTION

The non-communicable disease, diabetes mellitus, can be divided into two major types, viz. type 1 diabetes (T1D) and type 2 diabetes (T2D). T1D is characterized by β-cell autoimmunity. In T2D, the pancreatic β-cells produce insufficient insulin, and the peripheral tissues (liver, muscle and adipose tissue) are "resistant" to actions of insulin, i.e. glucose uptake is inefficient in these target tissues. The β-cells are also often destroyed in late stages of T2D making the patient dependant on insulin treatment. In diabetes patients, approximately 5-10% are type 1 and 90-95% are type 2 diabetic.

Major diabetic complications include retinopathy, cerebrovascular disease, coronary heart disease, neuropathy, peripheral vascular disease, ulceration and amputation. Thus diabetes affects several organs and tissues throughout the body. Factors that contribute to the development of diabetes include ethnicity (where certain population groups have an increased incidence of T2D, particularly if they have migrated), obesity, a high fat diet, the intrauterine environment, insulin resistance and specific candidate genes. The incidence of type 2 diabetes is increasing worldwide. Although genetic factors may play a role, life-style changes such as the adoption of a Western diet, high in fat, leads to obesity which can be a factor also contributing to the increase of this disease. Life-style factors, such as increased fat intake and reduced exercise, have been shown to be associated with obesity and insulin resistance (Lipman *et al.,* 1972; Lovejoy and DiGirolamo, 1992). In rats, high fat feeding induces a state of insulin resistance associated with diminished insulin-stimulated glycolysis and glycogen synthesis (Kim et al., 2000). This disease is a result of the peripheral insulin-responsive tissues, such as muscle and adipose tissue, displaying a significant decrease in response to insulin resulting in an increase in circulating glucose and fatty acids in the blood. The low response to insulin results in a decrease in glycolysis which in turn initiates gluconeogenesis and glycogenolysis in the liver, both of which are "switched off" by insulin under normal conditions. Pancreatic β-cells are able to cope with the initial insulin resistant phase by producing an excess of insulin and increasing the amount of insulin secreted (Pirol et al., 2004). The resulting hyperinsulinaemia to maintain normoglycaemia eventually brings about cell dysfunction (Khan, 2003) leading to overt diabetes. It is evident that type 2 diabetes is dependent on insults occurring both at peripheral as well as the cellular level (Khan *et al.,* 2000).

It is well established that insulin resistance and subsequent β-cell failure are major factors influencing the progression from normal glucose tolerance, through impaired glucose tolerance, to T2D. Lifestyle changes associated with the move from a rural to an urban area lead to an increase in obesity in urban black South Africans and this is associated with insulin resistance, which is another feature of T2D. To compensate for the insulin resistant state, β-cells produce more insulin and this leads to a higher demand on the already overworked β-cells which will result in β-cell exhaustion and ultimately β-cell failure.

There were an estimated 30M people with diabetes in the world in 1985. By 1995 the number had increased to 135M. The latest World Health Organization (WHO) estimate is that 300M people will have diabetes in 2025, an increase of 122%. This is in agreement with the International Diabetes Federation (IDF) estimation, in 2025, of 333M people with diabetes (6.3% prevalence), while 472M will be diagnosed with impaired glucose tolerance (IGT; 9% prevalence). There will be an estimated increase of 42% from 51 M to 72M, for the developed world (where there is an increase in the incidence of overweight and obese individuals, increasing their risk for becoming diabetic) and an increase of 170% from 84M to 228M for developing countries (due to a myriad of factors including dietary changes, increased physical activity and rapid urbanization). Thus while diabetes was previously considered a western life style disease affecting people in the developed countries, current trends suggest that by 2025 over 75% of all people with diabetes will be in the developing world. Another contributing factor to the increased incidence of diabetes in the developing world is developmental programming. In many parts of the developing world, people are often exposed to a poor diet (undernutrition) *in utero* followed by overnutrition postnatally which has been shown to predispose individuals to developing T2D. Furthermore, a total of 1.1 billion people are currently overweight, and 320M people are obese (IDF). This emphasizes the huge global economic burden of obesity, and as obesity is a major risk factor for developing diabetes, this may potentially further exacerbate the already huge economic burden associated with diabetes.

Conservative estimates for South Africa, based on minimal data, predict an increase from 5.6M in 2000 to over 8M in 2010. The largest increase is most likely to be in the black populations due to urbanisation, since this is accompanied by lifestyle and dietary changes from a low fat to a high fat diet. Figures in 1998 showed urban black South Africans to have an escalating incidence of T2D with the age-adjusted prevalence approaching 8% (Levitt, 1993), almost double the figure of 4.2% published in 1974 (Joffe & Seftel). In 1996 data for the top twenty causes of deaths in South Africa (Bradshaw et al, 1996) revealed diabetes to be the 10^{th} in males and 7^{th} in females. However, complications of diabetes, such as ischaemic heart disease and other cardiovascular and kidney complications contribute significantly to the number of deaths and adjusted figures could place diabetes in the top three or four causes of death in South Africa. The incidence of T2D is greater than HIV in SA, thus T2D warrants national attention.

Diabetes is an expensive disease and, although information on the cost of treating diabetics in South Africa is not available, there are many indirect costs. These include a reduction in the quality of life, the ability to contribute to the community and the workforce and its effect on the economy. This is exacerbated by an increased cost to Medical Aid Schemes resulting in increased Medical Aid premiums. Diabetes also affects the economy directly. In the absence of diabetes related financial data in South Africa, published data from the UK show that with more than 1.5M adults in the UK currently diagnosed with diabetes and its complications, the total National Health Service (NHS) cost is £5.2 billion each year, which is 9% of the total NHS budget. In the USA, the estimated direct costs are US$ 44 billion and, with loss of productivity, this figure increased to US$ 98 billion. Similar figures are available for many other countries.

T2D is diagnosed by raised levels of plasma glucose. However, our previous research has shown that by the time blood glucose levels increase, serious damage has already occurred in the cardiovascular system and the pancreas. Following diagnosis of diabetes by raised blood glucose levels, therapies such as diet and exercise and/or available medication can result in a temporary improvement in plasma glucose levels but cannot halt the progression of the disease. The rate of failure of these therapies is associated with the rate of continuing β-cell decline. Current treatment involves insulin injection or stimulating insulin release and/or action by medication.

There are many theories for explaining the impairment of insulin production by the pancreas that leads to the diabetic condition. Reference is made to two papers: "Mechanisms of pancreatic beta-cell destruction in type I diabetes" by Nerup J, Mandrup-Poulsen T, Molvig J, Helqvist S, Wogensen L, Egeberg J. published in Diabetes Care. 1988; 11 Suppl 1:16-23; and the second entitled "Autoimmune Imbalance and Double Negative T Cells Associated with Resistant, Prone and Diabetic Animals", Hosszufalusi, N., Chan, E., Granger, G., and Charles, M.; J Autoimmun, 5: 305-18 (1992). These papers show that inflammation of the pancreatic islets interrupts insulin production. Specifically, the insulin producing β-cells in the pancreatic islets are destroyed by immune attack. Such β-cell destruction is recognized as being due to attack by several types of immune cells including NK (natural killer) cells and double negative T-Lymphocytes.

Diabetes is considered to be insidious, since there is no known cure. Various treatments, however, have been used to ameliorate diabetes. For example, dietetic measures have been employed to balance the relative amounts of proteins, fats, and carbohydrates in a patient. Diabetes education and awareness programmes have also been implemented in several countries. In addition, diabetic conditions of moderate or severe intensity are treated by the administration of insulin. Also, prescription drugs such as "Glucoside" have been employed to rejuvenate impaired insulin production in adult onset diabetics. Other drugs are used to modulate the effectiveness of insulin. In any case, treatment of either juvenile or adult onset diabetes, has achieved only partial success. This is due to most agents targeting either improved beta-cell function or reducing insulin resistance, with the effect attenuating as the disease progressively worsens. Thus patients require the use (often daily) of a combination of agents to control the disease.

Biguanides, such as metformin, became available for treatment of type 2 diabetes in the late 1950s, and have been effective hypoglycaemic agents ever since (Vigneri and Goldfine, 1987). Little is known about the exact molecular mechanism of these agents. As an insulin sensitizer, metformin acts predominantly on the liver, where it suppresses glucose release (Goldfine, 2001). Metformin has also been shown to inhibit the enzymatic activity of complex I of the respiratory chain and thereby impairs both mitochondrial function and cell respiration, and in so doing decreasing the ATP/ADP ratio which activates AMP activated protein kinases causing catabolic responses on the short term and insulin sensitization on the long term (Brunmair et al., 2004; Tiikkainen et al., 2004). This drug has been proven effective in both monotherapy and in combination with sulfonylureas or insulin (Davidson and Peters, 1997). Diabetes in the young is a global phenomenon that is increasing in incidence. Some key transcription factors, important for beta-cell development, differentiation and function, are implicated in diabetes in the young. Some of these are direct targets of current therapeutic agents. The cost of current diabetic drugs is very high and the development of more affordable alternative therapies would be an advantage. The global burden of T2D is huge. Strategic action is required to endure affordable diabetes treatment to improve the quality of life of those individuals affected. This is particularly true for the developing world. It is for this reason that scientists are investigating the efficacy of indigenous plant extracts in their own country.

Honeybush (*Cyclopia spp.*) is indigenous to the Western and Eastern Cape provinces of South Africa. It is used to make a herbal tea, having a pleasant, mildly sweet taste and aroma, somewhat like honey. In the 1990s interest in honeybush as a herbal tea and as a substitute for ordinary tea (*Camellia sinensis*) was revived, both on the local and international markets. Most of the tea produced, is currently sold on the internationally market

International interest in honeybush is traced back to the tea trade of the Dutch and the British. A settlement, which eventually became Cape Town, was established in 1652 as a supply base for the Dutch East India Company that was trading in Indian tea and Southeast Asian spices. Botanists began cataloguing the rich flora of the cape soon after; the honeybush plant was noted in botanical literature by 1705. It was soon recognized by the colonists as a suitable substitute for ordinary tea, probably based on observing native practices. In 1814, the Cape Colony fell under Britsih rule, and English became the official language a few years later, helping to spread knowledge of South Africa to England and America. It was noted in 1871 (Smith, 1966) that the tea (called "bergtee") growing wild on the Langeberg and Swartberg Mountains, was extensively used as a beverage in those areas. In King's American Dispensatory of 1898, under the heading of tea, honeybush is already listed as a substitute, with reference to a report from 1881 indicating use of honeybush as a tea in the Cape Colony of South Africa. Early colonists also used a decoction of honeybush as an expectorant for chronic catarrh and pulmonary tuberculosis.Watt & Breyer-Brandwijk, 1932). It was also used as a restorative. According to Marloth (1925), honeybush was valued as a stomachic (digestive aid).

The plant is a shrub of the Fabaceae family (Leguminosae) that grows in the fynbos botanical zone (biome). It is a narrow region along the coast, bounded by mountain ranges. Fynbos is a vegetation type, characterized mainly by woody plants with small leathery leaves (fynbos is from the Dutch, meaning fine leaved plants).

The honeybush plant is easily recognized by its trifoliate leaves, single-flowered inflorescences, and sweetly scented, bright yellow flowers. The flowers have prominent grooves on the petals, a thrust-in (intrusive) calyx base, and two bracts fused at the base around the pedicel. The genus name *Cyclopia* alludes to the intrusive base of the calyx, which contributes to the flower's unique appearance. Honeybush plants have woody stems, a relatively low ratio of leaves to stems, and hard-shelled seeds. The most desirable components for the tea are the leaves and flowers, but the relatively tasteless stems are included in the product.

Most of the honeybush tea is still collected from wild populations, but cultivation has become necessary with the rapid growth of the industry. Commercial supplies of honeybush are mainly obtained from *Cyclopia intermedia* and to *Cyclopia subternata,* though there are about 2 dozen species of *Cyclopia* identified in this narrow region of South Africa. Most of the species have very limited distribution ranges and unique habitat preferences. Some are restricted to mountain peaks, perennial streams, marshy areas, shale bands, or wet southern slopes. Some of the species, such as *Cyclopia maculata, Cyclopia genistoides,* and *Cyclopia sessiliflora,* have been used for home consumption. It appears that all the *Cyclopia* species are suitable for making tea, but the taste quality can vary, and some species exist in very small quantities.

Leaf shape and size differ among the species, but most are thin, needle-like to elongated leaves. All the species are easily recognized in the field during flowering as they are covered with the distinctive, deep-yellow flowers, which have a characteristic sweet honey scent. Traditionally, the tea was harvested during flowering- either in early Autumn or late Spring-depending on the flowering period of the species. However, with the larger demand for products, harvesting now takes place during summer.

The production of honeybush in South Africa has grown significantly in recent years. In 1999, approximately 50 tons of the plant was exported, growing to 300 tons by 2005, with Germany the major market. Most of the tea is exported, but there has also been a substantial growth in domestic consumption. The tea is mainly sold as the traditional "fermented" (oxidized) product, but small quantities of green ("unfermented") honeybush, since its first commercial production in 2001, is also sold on the local and international market.

Honeybush tea is made as a simple herbal infusion. One of its early recognized benefits as a tea substitute is its lack of caffeine, which makes it especially suited for nighttime consumption and for those who experience nervousness and want to avoid ordinary tea. As a result, it had a reputation as a calming beverage, though it may not have any specific sedative properties. It also has a lower tannin content than ordinary tea, so it doesn't make a highly astringent tea, which can be a problem with some grades of black or green tea or when ordinary tea is steeped too long.

The traditional use of the tea for treating coughs may be explained, in part, by its content of pinitol, a modified sugar (a methyl group replaces hydrogen in one position of glucose;) that is similar to inositol. Pinitol, named for its major source, pine trees, is also found in the leaves of several legume plants; it is an expectorant. Pinitol is also of interest for apparent blood-sugar lowering effects, as demonstrated in laboratory animal studies (it may increase the effects of insulin), and is being considered as a drug for diabetes. Honeybush also contains xanthones, flavanones, flavones, isoflavones, coumestans and 4-hydroxycinnamic acid, with qualitative and quantitative differences in the phenolic composition between *Cyclopia* species. Mangiferin and hesperidin are the major monomeric polyphenols present in *Cyclopia* species. The polyphenols serve as antioxidants and may help protect blood lipids. The isoflavones and coumestans are classified as phytoestrogens, used in the treatment of menopausal symptoms, an application for which honeybush has recently been evaluated..

As a result of its adipogenic effect, insulin has the undesirable effect of promoting obesity in patients with type 2 diabetes. (Moller, D. E. (2001) Nature 414:821-827). Unfortunately, other anti-diabetic drugs, including metformin, which are currently being used to stimulate glucose transport in patients with type 2 diabetes also possess adipogenic activity. Thus while current drug therapy may provide reduction in blood sugar, it often promotes obesity. Accordingly, new compositions and methods for treating hyperglycemia are desirable. Compositions that stimulate glucose uptake without generating concomitant adipogenic side effects are especially desirable.

MCKay et al (2007) provides a review of the bioactivity Rooibos (Aspalathus linearis) and honeybush (Cyclopia intermedia) without disclosing the antidiabetic potential of these plants.

Hiroyuki et al (1998) discloses new antidiabetic compounds, including mangiferin. The authors that oral administration of mangiferin at doses of 10, 30 and 90 mg/kg are useful for the treatment of diabetes.

### SUMMARY OF THE INVENTION

In accordance with the present invention novel and useful compositions derived from honeybush for treating diabetes are provided.

It has surprisingly been found that the plant extract of the present invention exhibit a superior antidiabetic effect when administered in an amount from 0.1 milligram to 25 milligrams per kilogram body weight.

Accordingly, there is provided a plant extract and a pharmaceutical preparation including the plant extract in dosage unit form for oral administration, for use in the treatment of diabetes. The plant extract is obtainable by a method comprising the steps of:
(a) providing *Cyclopia* plants or portions thereof,
(b) combining said plants or portions thereof with a nontoxic solvent appropriate for solubilizing said plant extract, and heating the solvent to a temperature between 60 °C and 95°C;
(c) recovering said plant extract, and
(d) drying;
wherein said therapeutically effective amount is from 0.1 milligram to 25 milligrams per kilogram body weight.

The treatment of the present invention was discovered because the inventors found that an aqueous extract of honeybush (*Cyclopia* species; Fynbos) was effective in controlling blood sugar. For the medical use in accordance with the present invention the plant is gathered, dried, and combined with a solvent such as water and/or an alcohol, preferably ethanol.

Accordingly, in a first aspect the present disclosure relates to an anti-diabetic composition comprising an aqueous extract of plants of the genus *Cyclopia,* preferably *Cyclopia genistoides, Cyclopia subternata, Cyclopia intermedia, Cyclopia sessiflora, Cyclopia maculata, Cyclopia longifolia, Cyclopia plicata, Cyclopia pubescens, Cyclopia bauxiflolia, Cyclopia meyeriana* and/or combinations thereof.

The present inventors have found that administering an extract of the plant genus *Cyclopia* allows for treatment of diabetes, and in particular of early stages of diabetes, also referred to as pre-diabetic states.

Thus, the present invention relates to compositions derived from the plant genus *Cyclopia* and methods for treating subjects who are hyperglycemic, particularly subjects with Type II diabetes as well as diabetic subjects who are overweight. In a preferred embodiment the present invention provides a *Cyclopia* plant extract for treating subjects who are hyperglycemic.

The treatment of the present invention was discovered because the inventors found that a steam or aqueous extract of *Cyclopia* was effective in controlling blood sugar. For use the plant is gathered, dried, and combined with boiling water. The extract is then taken orally by a patient on a periodic basis. *Cyclopia* is known to be rich in flavonoids and other secondary plant products.

### BRIEF DESCRIPTION OF THE FIGURES

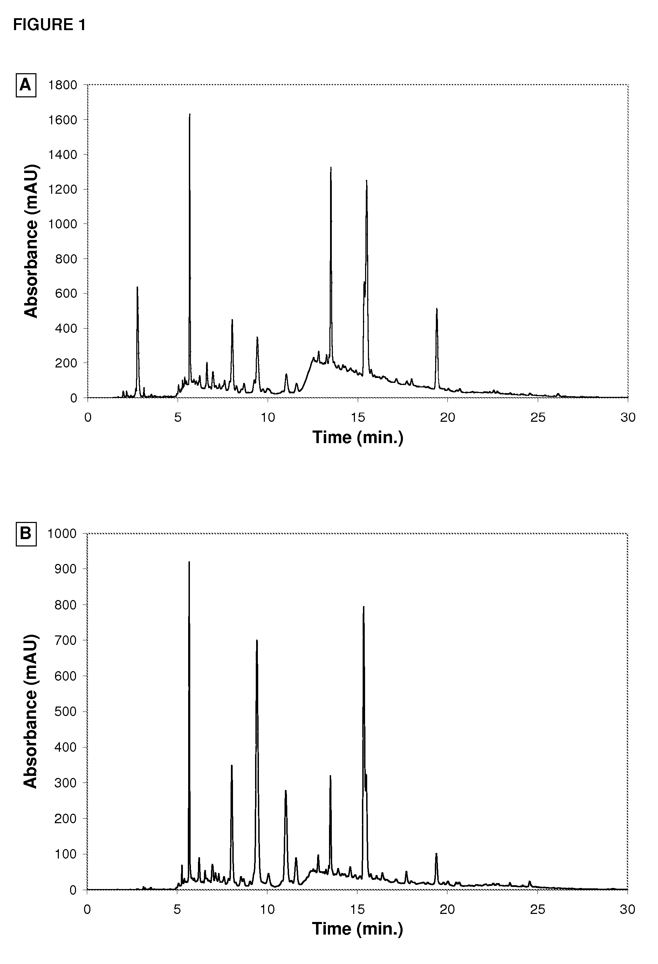

**Figure 1****.** Shows an HPLC fingerprint of aqueous extracts of *Cyclopia* plant material used for preparation of GMP ARC137 (A = chromatogram at 288 nm; B = chromatogram at 320 nm).

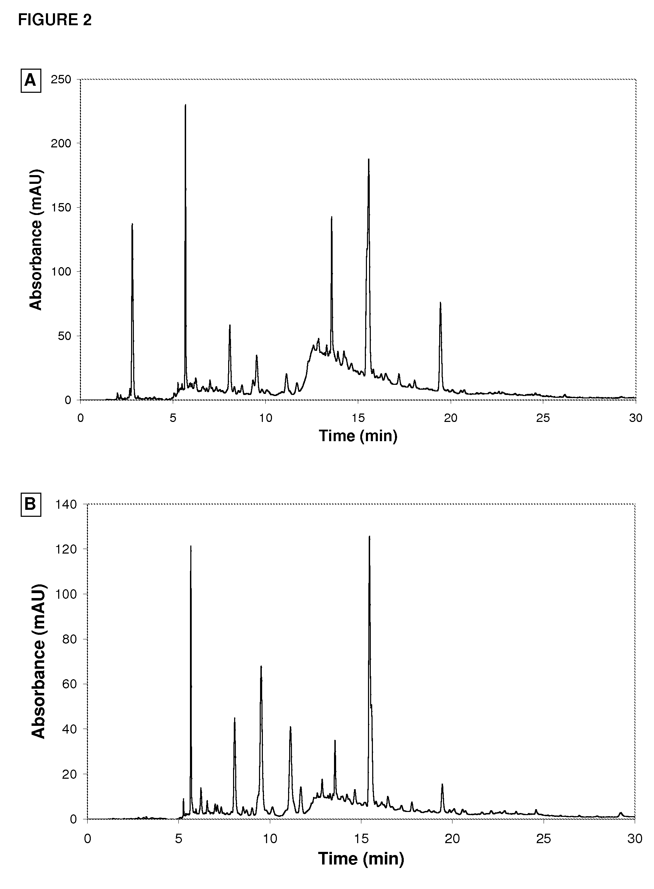

**Figure 2****.** Shows an HPLC fingerprint of GMP ARC137 (A = chromatogram at 288 nm; B = chromatogram at 320 nm).

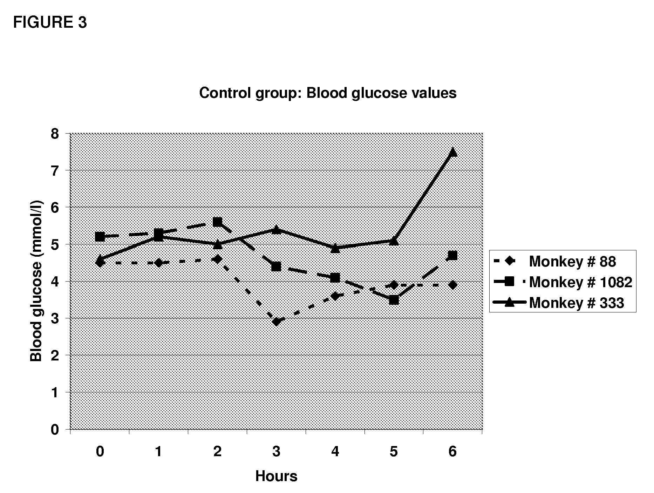

**Figure 3****.** Shows hourly blood glucose values of the three control monkeys plotted over a 6 hour monitoring period. The baseline values (0 hours) represent the fasting blood glucose values before the maintenance diet bolus was fed to the monkeys.

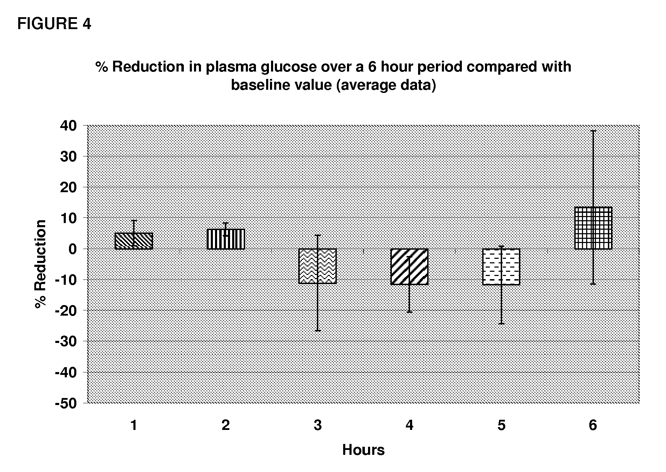

**Figure 4****.** Shows the mean percentage blood glucose decline or increase as calculated from the baseline blood glucose level in the control group. Blood glucose values were maintained around the baseline values with slightly lower values seen at three, four and five hours after the monkeys received a 70 g maintenance diet portion.

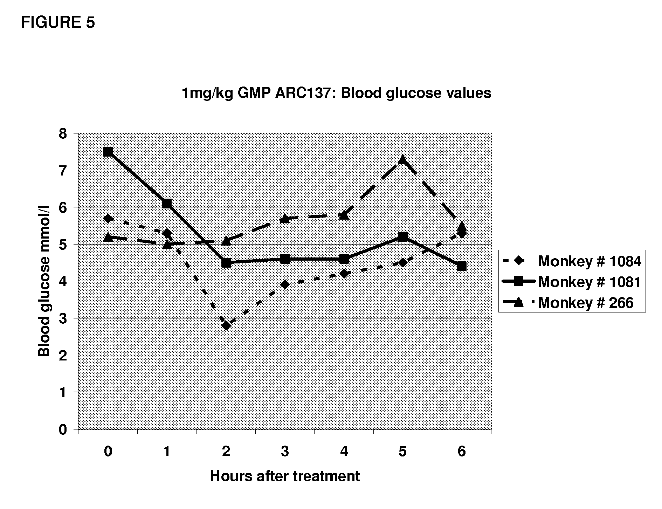

**Figure 5****.** Shows blood glucose values of the three monkeys in the experimental group receiving 1 mg/kg GMP ARC137 plotted over a 6 hour monitoring period. The baseline values (0 hours) represent the fasting blood glucose values before the maintenance diet bolus containing 1 mg/kg GMP ARC137 was given to the monkeys.

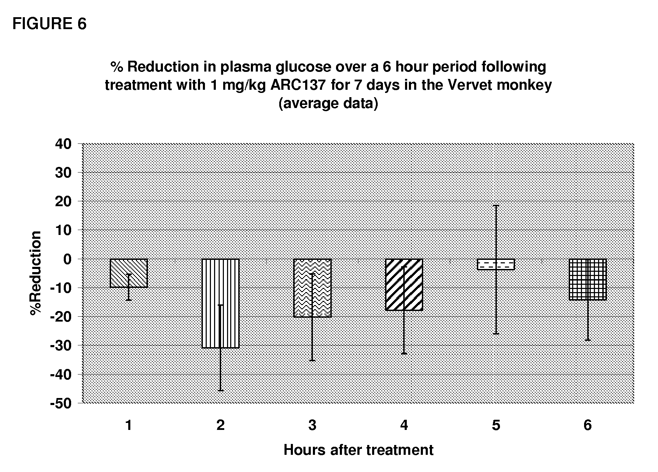

**Figure 6****.** Shows the mean percentage blood glucose decline or increase as calculated from the baseline blood glucose level. The highest percentage reduction of blood glucose occurred at 2 hours after receiving 1 mg/kg GMP ARC137. Marked lower glucose levels were still maintained at three and four hours.

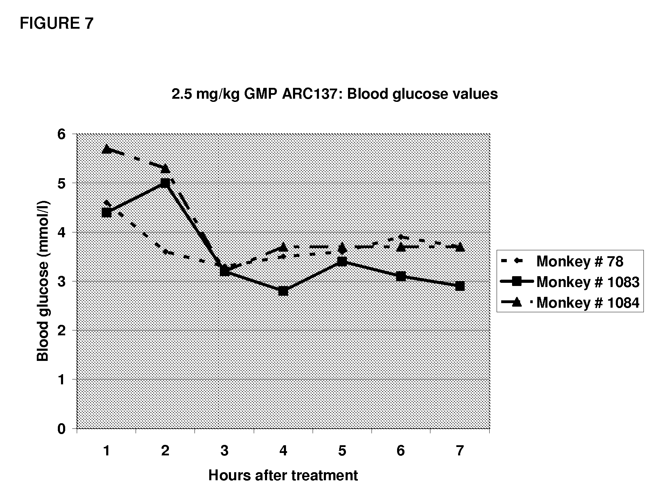

**Figure 7****.** Shows blood glucose values of the three monkeys in the experimental group receiving 2.5 mg/kg GMP ARC137 plotted over a 6 hour monitoring period. The baseline values (0 hours) represent the fasting blood glucose values before the maintenance diet bolus containing 2.5 mg/kg GMP ARC137 was given to the monkeys.

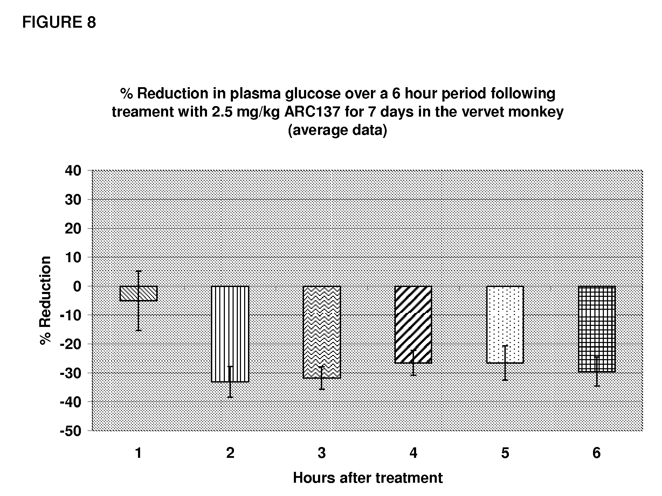

**Figure 8****.** Shows the mean percentage blood glucose decline or increase as calculated from the baseline blood glucose level. The highest percentage reduction of blood glucose occurred at 2 hours after receiving 1 mg/kg GMP ARC137. Thereafter these lower levels were maintained for the full six hours of the monitoring period.

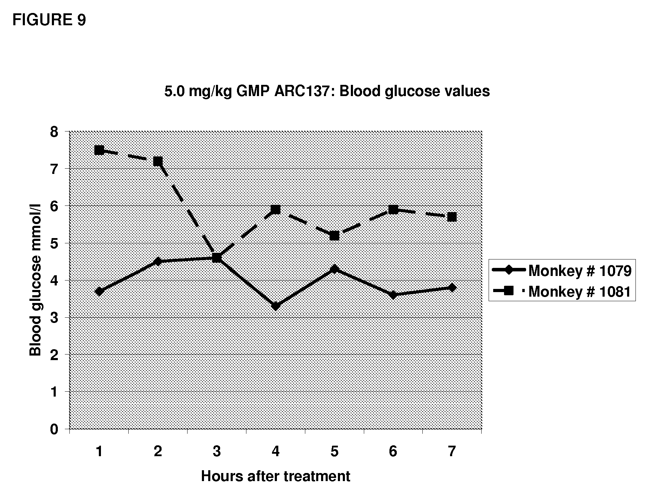

**Figure 9****.** Shows blood glucose values of the three monkeys in the experimental group receiving 5 mg/kg GMP ARC137 plotted over a 6 hour monitoring period. The baseline values (0 hours) represent the fasting blood glucose values before the maintenance diet bolus containing 5.0 mg/kg GMP ARC137 was given to the monkeys.

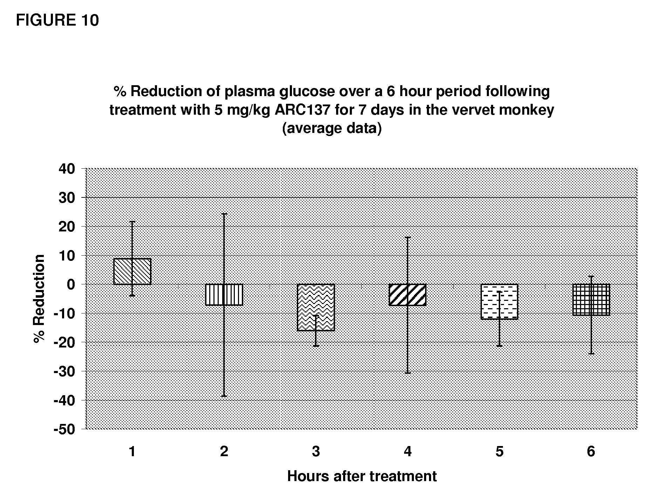

**Figure 10****.** Shows the mean percentage blood glucose decline or increase as calculated from the baseline blood glucose levels. At two hours after receiving 5 mg/kg GMP ARC137 mean blood glucose percentages dropped, albeit a small reduction, to below that of the baseline and maintained these levels for the remaining four hours of the monitoring period.

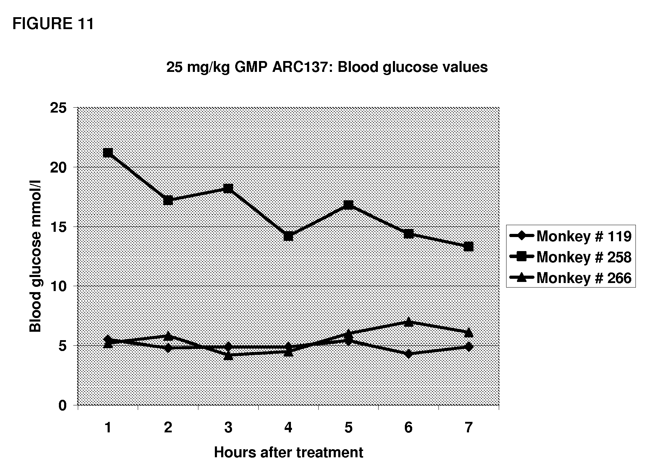

**Figure 11****.** Shows blood glucose values of the three monkeys in the experimental group receiving 25 mg/kg GMP ARC137 plotted over a 6 hour monitoring period. The baseline values (0 hours) represent the fasting blood glucose values before the maintenance diet bolus containing 2.5 mg/kg GMP ARC137 was given to the monkeys.

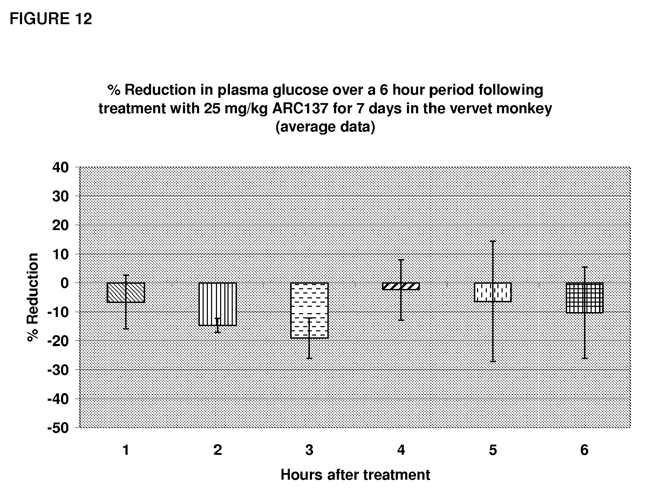

**Figure 12****.** Shows the mean percentage blood glucose decline or increase as calculated from the baseline blood glucose level. After receiving the 25 mg/kg GMP ARC137 blood glucose percentages were marginally lower than the baseline levels at all hourly time points of the monitoring period. The highest percentage reduction of blood glucose percentage occurred at 3 hours.

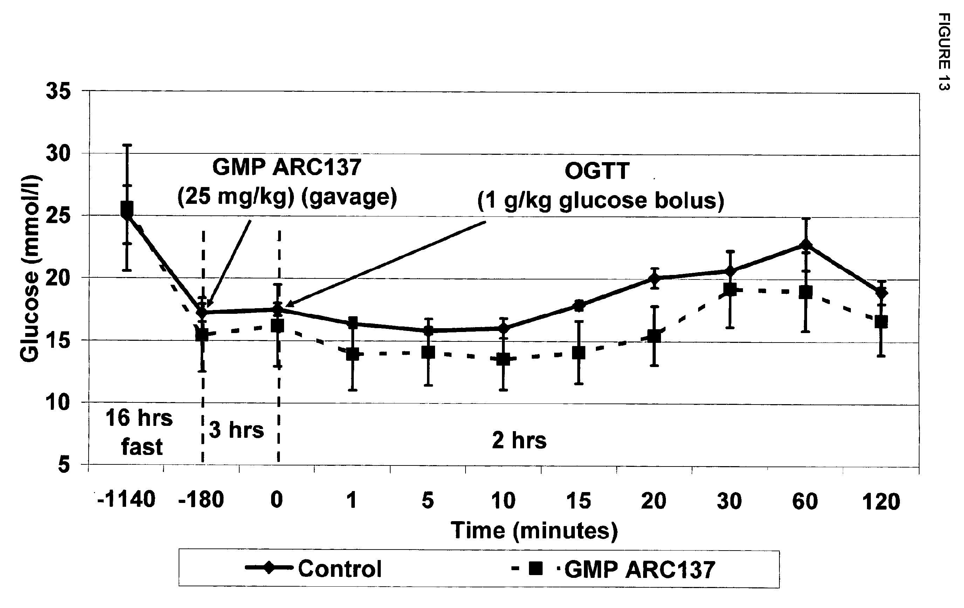

**Figure 13****.** Shows the results of the OGTT (1 g/kg glucose) performed three hours after a single 25 mg/kg GMP ARC137 oral dose. The resulting plasma glucose levels of the STZ rats were lower when compared to control glucose values at all the time points taken.

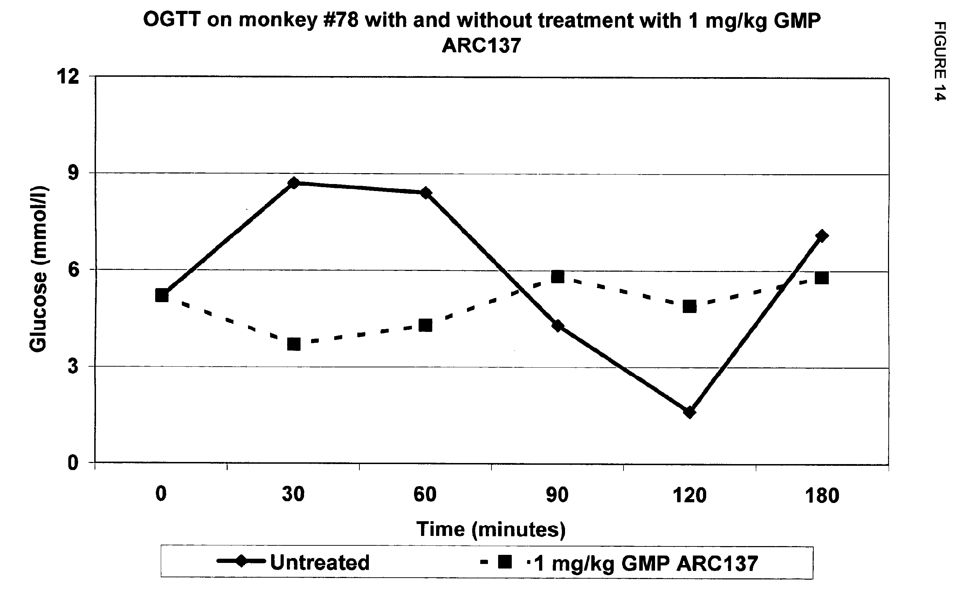

**Figure 14****.** Shows the results of the OGTT (1.75 g/kg glucose) performed over three hours, after a single 1 mg/kg GMP ARC137 oral dose. The resulting plasma glucose levels of the monkey #78 were lower when compared to untreated monkey glucose values for the first 90 minutes.

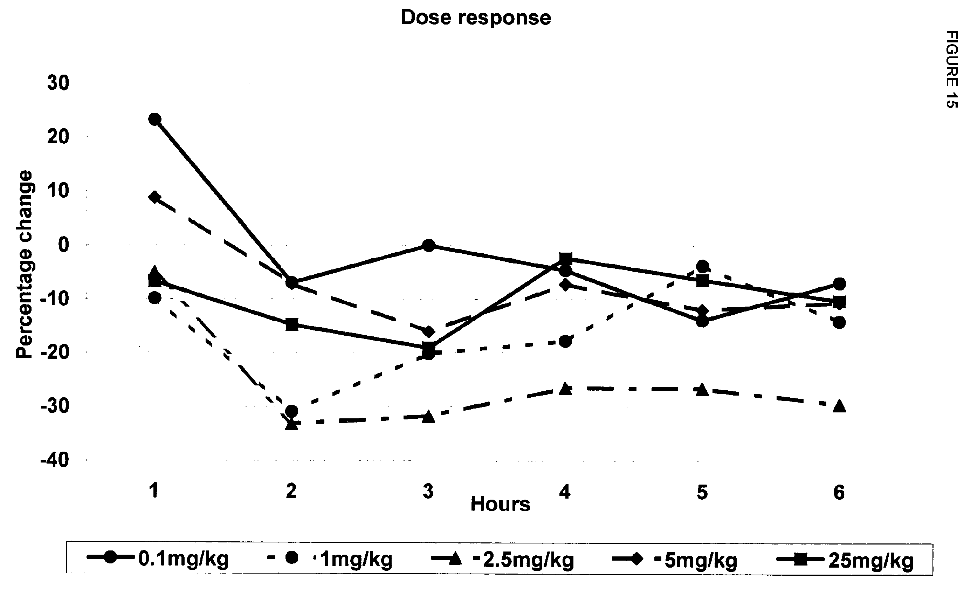

**Figure 15****.** Shows % reduction in plasma glucose over a 6 hour period following treatment with different dosages of the extract (ARC137) for 7 days in Vervet monkey.

### DETAILED DESCRIPTION OF THE INVENTION

The following description is provided to enable any person skilled in the art to make and use the invention and sets forth the best modes contemplated by the inventor of carrying out his invention. Various modifications, however, will remain readily apparent to those skilled in the art, since the general principles of the present invention have been defined herein specifically to provide treatment of both insulin-dependent and non-insulin dependent diabetes through the administration of flavonoids particularly through the administration of a plant extract.

### Definitions

The term "diabetes mellitus" or "diabetes" means a disease or condition that is generally characterized by metabolic defects in production and utilization of glucose which result in the failure to maintain appropriate blood sugar levels in the body. The result of these defects is elevated blood glucose, referred to as "hyperglycemia." Two major forms of diabetes are Type 1 diabetes and Type 2 diabetes. As described above, Type 1 diabetes is generally the result of an absolute deficiency of insulin, the hormone which regulates glucose utilization. Type 2 diabetes often occurs in the face of normal, or even elevated levels of insulin and can result from the inability of tissues to respond appropriately to insulin. Most Type 2 diabetic patients are insulin resistant and have a relative deficiency of insulin, in that insulin secretion can not compensate for the resistance of peripheral tissues to respond to insulin. In addition, many Type 2 diabetics are obese. Other types of disorders of glucose homeostasis include impaired glucose tolerance, which is a metabolic stage intermediate between normal glucose homeostasis and diabetes. The guidelines for diagnosis for Type 2 diabetes and impaired glucose tolerance have been outlined by the American Diabetes Association (see, e.g., The Expert Committee on the Diagnosis and Classification of Diabetes Mellitus, Diabetes Care, (1999) Vol 2 (Suppl 1): S5-19).

The term "symptom" of diabetes, includes, but is not limited to, polyuria, polydipsia, and polyphagia, hyperinsulinemia, and hyperglycemia as used herein, incorporating their common usage. For example, "polyuria" means the passage of a large volume of urine during a given period; "polydipsia" means chronic, excessive thirst; "polyphagia" means excessive eating, and hyperinsulinemia means elevated blood levels of insulin. Other symptoms of diabetes include, for example, increased susceptibility to certain infections (especially fungal and staphylococcal infections), nausea, and ketoacidosis (enhanced production of ketone bodies in the blood).

### Dosage

The honeybush (*Cyclopia* ssp.) plant extract is administered to the subject in a therapeutically effective amount. As used herein, the term "therapeutically effective amount" means the total amount that is sufficient to show a meaningful benefit, i.e., a reduction in the subject's blood glucose levels. The dosages of the plant extract needed to obtain a meaningful result, can be determined in view of this disclosure by one of ordinary skill in the art by running routine trials with appropriate controls. Comparison of the appropriate treatment groups to the controls will indicate whether a particular dosage is effective at reducing the subject's blood glucose levels. When orally administered the extract should be in doses of at least 0.1 mg/kg body weight, preferably at least 1 mg/kg, more preferably at least 2.5 mg/kg, even more preferably at least 5 mg/kg, most preferably at least 25 mg/kg, such as at least 50 mg/kg, at least 75 mg/kg, at least 100 mg/kg, at least 250 mg/kg, and at least 500 mg/kg.

The amount of the plant extract required will depend upon the nature and severity of the condition being treated, and on the nature of prior treatments which the subject has undergone. Ultimately, the dosage will be determined using clinical trials. Initially, the clinician will administer doses that have been derived from animal studies. An effective amount can be achieved by one administration of the composition. Alternatively, an effective amount is achieved by multiple administration of the composition to the subject. In vitro, the biologically effective amount, i.e., the amount sufficient to induce glucose uptake, is administered in two-fold increments, to determine the full range of activity. The efficacy of oral, subcutaneous and intravenous administration is determined in clinical studies. Although a single administration of the extract may be beneficial, it is expected that multiple doses will be preferred.

### Delivery

Administration of the honeybush plant extract preferably is by oral administration. Although less preferred, the extract may also be administered by injection. Formulations suitable for oral administration may be presented as discrete units such as capsules, cachets, tablets, boluses or lozenges, each containing a predetermined amount of the active compound; as a powder or granules; or in liquid form, e.g., as an aqueous solution, suspension, syrup, elixir, emulsion, dispersion, or the like. The extract may be administered in the form of pills (powder or concentrated liquid in capsules), or powder form (e.g. dried powder but pressed into grains) that can be consumed after putting into water (similar to drinking tea or coffee).

Formulations suitable for parenteral administration conveniently comprise a sterile preparation of the active compound in, for example, water for injection, saline, a polyethylene glycol solution and the like, which is preferably isotonic with the blood of the recipient. Useful formulations also comprise concentrated solutions or solids containing the honeybush plant extract which upon dilution with an appropriate solvent give a solution suitable for parenteral administration.

In addition to the aforementioned ingredients, the formulations may further include one or more optional accessory ingredient(s) utilized in the art of pharmaceutical formulations, i.e., diluents, buffers, flavoring agents, colorants, binders, surface active agents, thickeners, lubricants, suspending agents, preservatives (including antioxidants) and the like.

The amount of the honeybush plant extract required to be effective for any indicated condition will, of course, vary with the individual mammal being treated and is ultimately at the discretion of the medical or veterinary practitioner. The factors to be considered include the condition being treated, the route of administration, the nature of the formulation, the mammal's body weight, surface area, age and general condition, and the particular extract to be administered. The total daily dose may be given as a single dose, multiple doses, e.g., two to six times per day, or by intravenous infusion for a selected duration. Dosages above or below the range cited above may be administered to the individual patient if desired and necessary.

The composition comprises a biologically effective amount of the honeybush plant extract, and, optionally, a relatively inert carrier. Many such carriers are routinely used and can be identified by reference to pharmaceutical texts. The acceptable carrier is a physiologically acceptable diluent or adjuvant. The term physiologically acceptable means a non-toxic material that does not interfere with the effectiveness of the analog. The characteristics of the carrier will depend on the route of administration and particular compound or combination of compounds in the composition. Preparation of such formulations is within the level of skill in the art. The composition may further contain other agents that either enhance the activity of the analog or complement its activity. The composition may further comprise fillers, salts, buffers, stabilizers, solubilizers, and other materials well known in the art.

Concerning the xanthones and flavonoids the following should be mentioned.

Mangiferin is a natural molecule found in honeybush. The molecule is classified as a xanthone.

Mangiferin has the following chemical structure: 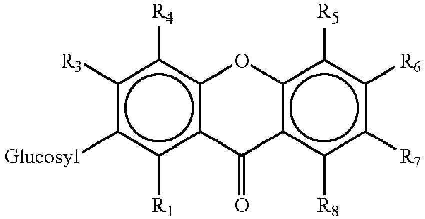 wherein R₁=R₃=R₆=R₇=OH and R₄=R₅=R₈=H.

Isomangiferin also exists naturally, having the following structure: 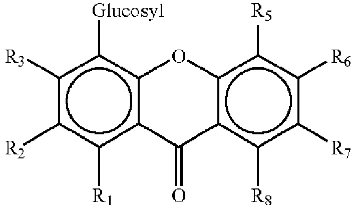 wherein R₁=R₃=R₆=R₇=OH and R₂=R₅=R₈=H.

Its derivatives of natural origin may have O-methyl groups (-OCH₃) or glucosyl groups (-C₆H₁₁O₆) at positions R₁, R₃, R₆ or R₇.

All the compounds formed by mangiferin, its isomers and its derivatives correspond to the following general formula I: 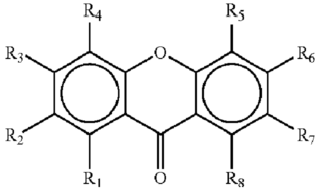 wherein R₁, R₂, R₃, R₄, R₅, R₆, R₇, R₈ are chosen from -H, -OH, -OCH₃ and a glucosyl radical.

Compounds of formula I may be obtained by different means, including extracting from plant material (honeybush).

Alternatively compounds of formula I may be obtained for example by purifying extracts of all or part of plants known to comprise such compounds using any extraction or purification process (e.g. extraction with a polar solvent such as water, an alkanol, or mixture of these solvents, subsequent purification by crystallization or any other method known to those skilled in the art). Some of these methods are described for example in the patent published under number FR-A-2 486 941.

The compounds may also be obtained chemically or enzymatically. In this connection, methods are described inter alia in the following two articles: Bhatia-V-K et al., Tetrahedron Lett. (14), p. 1741-2 and Nott-P-E, Phytochemistry, Vol. 6(11), p. 1597-9.

Hesperidin is also a natural molecule found in honeybush. Hesperidin (7-[[6-O-(6-deoxy-α-L-mannopyranosyl)-β-D-glucopyranosyl]oxy]-2,3-dihydro-5-hydroxy-2-(3-hydroxy-4-methoxyphenyl)-4H-1-benzopyran-4-on; as well as its aglycone hesperetin are contemplated by the present inventors. The inventors has also contemplated equivalent flavone/flavanone derivatives of the general formula II

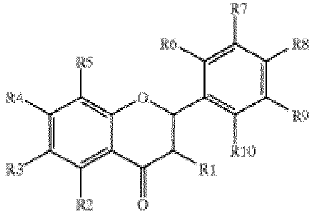

in which independent of one another
R1 is H, OH or -O-Gly;
R2, R6, R9 and R10 can be the same or different and H, OH, alkoxy, hydroxyalkoxy or C₃-C₇-cycloalkoxy;
R3 H, C₁-C₄alkyl or C₃-C₇-cycloalkoxy;
R4 H, OH, -O-Gly, alkoxy, hydroxyalkoxy or C₃-C₇-cycloalkoxy;
R5 H C₁-C₄-alkyl, OH, alkoxy, hydroxyalkoxy or C₃-C₇-cycloalkoxy;
R7 and R8 can be the same or different and H, OH, alkoxy, hydroxyalkoxy, C₃-C₇-cycloalkoxy, (thio(C₁-C₄-)alkyl or -NR11R12; and
R11 and R12 can be the same or different and can mean H or C₁-C₄-alkyl; and wherein Alkoxy and hydroxyalkoxy can contain a straight chain or branched alkyl group with 1-18 C-atoms; and wherein
Gly can be present or absent and designate a mono- or oligoglycidic residue;

Preferred flavone/flavanone derivatives of the general formula II are, for example:
1.) Rutin, (Rutoside; quercetin-3-rutinoside, 3-[[6-O-(6-deoxy-α-L-mannopyranosyl)-beta-D-glucopy-ranosyl]oxy]-2-(3,4-dihydroxyphenyl)-5,7-dihydroxy-4H-1-benzopyran-4-on); as well as its aglycone
2.) O-β-hydroxyethylrutoside, a mixture of mono-, di-, tri- and tetrahydroxyethyl derivatives of rutin; or their algycons; as well as the main component of O-β-hydroxyethylrutoside, namely
3.)Troxerutin-(2[3,4-bis(2-hydroxyethoxy)phenyl]-3-[[6-O-(6-deoxy-α-L-mannopyranosyl)-β-D-glucopyranosyl]oxy]-5-hydroxy-7-(2-hydroxyethoxy)-4H-1-benzopyran-4-on); as well as its aglycone;
4.) Monoxerutin-(2-[3,4-bishydroxyphenyl]-3-[[6-O-(6-deoxy-α-L-mannopyranosyl)-β-D-glucopyranosyl]oxy]-5-hydroxy-7-(2-hydroxyethoxy)-4H-1-benzopyran-4-on); as well as its aglycone;
5.) α-glucosylrutin;
6.) Naringin 7-[[2-O-(6-deoxy-α-L-mannopyranosyl)-β-D-glucopyranosyl]oxy]-2,3-dihydro-5-hydroxy-2-(4-hydroxyphenyl)-4H-1-benzopyran-4-on); as well as its aglycone naringenin;
7.) Hesperidin (7-[[6-O-(6-deoxy-α-L-mannopyranosyl)-β-D-glucopyranosyl]oxy]-2,3-dihydro-5-hydroxy-2-(3-hydroxy-4-methoxyphenyl)-4H-1-benzopyran-4-on; as well as its aglycone hesperetin;
8.) Diosmin (3'5,7-trihydroxy-4methoxyflavone-7-rhamnoglucoside; as well as its aglycone diosmetin;
9.) dihydrorobinetin (3,3',4',5',7-pentahydroxyflavanone);
10. Taxifolin (3,3',4',5,7-pentahydroxyflavanone);
11. Eriodictin (3',4',5,7,-tetrahydroxyflavanone-7-rhamnoside); as well as its aglycone eriodictyol;
12. Flavanomarein (3',4',7,8-tetrahydroxyflavanone-7-glucoside); as well as its aglycone;
13. Isoquercetin (3,3',4',5,7-pentahydroxyflavanone-3-(β-D-pyranoside); as well as its aglycone;
14. Leucocyanidin (3,3',4,4',5,7-hexahydroxyflavan);
15. Cyrtominetin (6-8-dimethyl-3',4',5,7-tetrahydroxyflavanone); 16. 6,8-dimethyl-5,7-dihydroxy-4'-thiomethylflavanone;
17. 6,8-dimethyl-4',5,7-trihydroxy-3'-methoxyflavanone; or
18. 6,8-dimethyl-5,7-(dihydroxy-4'-dimethylamino)-flavanone.

Moreover eriocitrin, narirutin and scolymoside are applicable (see also Ferreira et al., 1998, Kamara et al., 2003; Kamara et al., 2004).

Different species of honeybush contain these, or similar flavonoids, albeit in different proportions. Experiments with diabetic test animals (rats) were carried out. The honeybush extract was effective in controlling blood glucose in these model systems. Further, the administration of synthetic versions of the flavonoids were also effective at lowering glucose levels. However, there is some indication that a combination of mangiferin with the other flavonoids, especially hesperidin, results in an enhanced (synergistic) effect in that blood glucose can be maximally lowered with a lower overall flavonoid dose. The effect seems most pronounced when the molar concentration of mangiferin is at least twice that of hesperidin.

As hereinbefore and hereafter mentioned: "diabetes" preferably refers to non-insulin dependent diabetes (type 2); "anti-diabetic" means the activity useful for the "treatment" of "diabetes", which includes the prevention of the development of diabetes, and/or the treatment of established diabetes; it also includes the prevention of the causes of diabetes, and/or the decrease or disappearance of its symptoms and/or consequences.

In particular, it has been found that compounds mentioned herein have at least the following double therapeutic effect:
i) the prevention of diabetes, since the compounds can treat impaired glucose tolerance; and
ii) the actual treatment of established diabetes since the compounds can decrease the blood glucose level.

Preferably, the extract comprises as an active ingredient a compound having the essential features of mangiferin and/or a pharmaceutically acceptable salt or prodrug thereof. More preferably the extract also comprises as an active ingredient a compound having the essential features hesperidin. Most preferably the molar concentration of mangiferin is at least twice that of hesperidin.

According to a further aspect, the invention also concerns the said extract for use as a medicament having anti-diabetic activity.

The invention also extends to a pharmaceutical composition having anti-diabetic activity comprising an effective quantity of the said extract; and to mangiferin and hesperidin having anti-diabetic activity.

According to a still further aspect, the disclosure also concerns the use of the said extract in the manufacture of a foodstuff or beverage to have an anti-diabetic effect when ingested. The said foodstuff or beverage comprising an effective quantity of the said extract to have an anti-diabetic effect when ingested is also part of the present invention. Preferably, the said extract comprises as an active ingredient mangiferin and/or a pharmaceutically acceptable salt or prodrug thereof.

According to a further aspect, the invention also concerns the said extract for use as a medicament having anti-diabetic activity.

The active ingredient may be an extract from a plant of the genus *Cyclopia*, or a compound having the structure (mangiferin): 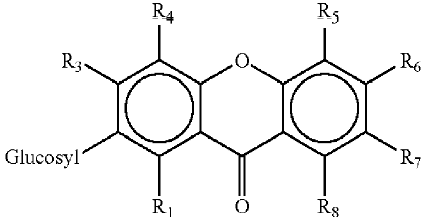 wherein R₁=R₃=R₆=R₇=OH and R₄=R₅=R₈=H,
alternatively the following structure: 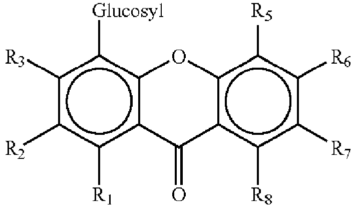 wherein R₁=R₃=R₆=R₇=OH and R₂=R₅=R₈=H.

Their derivatives of natural origin may have O-methyl groups (-OCH₃) or glucosyl groups (-C₆H₁₁O₆) at positions R₁, R₃, R₆ or R₇.

All the compounds formed by mangiferin, its isomers and its derivatives correspond to the following general formula I: 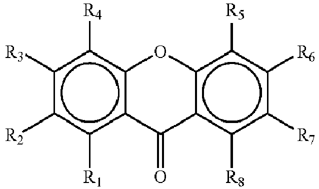 wherein R₁, R₂, R₃, R₄, R₅, R₆, R₇, R₈ are chosen from -H, -OH, -OCH₃ and a glucosyl radical.

These compounds are either extracted from a plant of the genus *Cyclopia* or prepared synthetically or a derivative thereof.

The plant may be of the species *Cyclopia subternata.*

Preferably, the compounds are prepared in pharmaceutically acceptable dosage forms. The anti-diabetic composition or formulation may consist of the anti-diabetic agent admixed with a pharmaceutical excipient, diluent or carrier. Other suitable additives, including a stabilizer and such other ingredients as may be desired may be added.

The extract having anti-diabetic activity according to the invention may be prepared in accordance with the following process. The process for preparing an extract of a plant of the genus *Cyclopia* comprising a anti-diabetic agent includes the steps of treating collected plant material with a solvent to extract a fraction having anti-diabetic activity, separating the extraction solution from the rest of the plant material, removing the solvent from the extraction solution and recovering the extract. The extract so recovered may be further purified, e.g. by way of suitable solvent extraction procedures.

The extract may be prepared from plant material such as the leaves, stems and roots of said plants of the genus *Cyclopia.* In one application of the invention, the anti-diabetic extract is obtained from the species *Cyclopia subternata.*

The plant material may be homogenised in the presence of a suitable solvent, for example, a methanol/methylene chloride solvent, by means of a device such as a Waring blender. The extraction solution may then be separated from the residual plant material by an appropriate separation procedure such as, for example, filtration or centrifugation. The solvent may be removed by means of a rotary evaporator, preferably in a water bath at a temperature of 60° C.

The separated crude extract may then be further extracted with methylene chloride and water before being separated into a methylene chloride extract and a water extract. The methylene chloride extract may have the solvent removed preferably by means of evaporation on a rotary evaporator and the resultant extract may be further purified by way of a methanol/hexane extraction. The methanol/hexane extraction product may then be separated to yield a methanol extract and a hexane extract. The methanol extract may be evaporated to remove the solvent in order to yield a partially purified active extract.

The partially purified active extract may be dissolved in methanol, and may be further fractionated by column chromatography, employing silica gel as an adsorption medium and a chloroform/30% methanol mixture as an eluent. A plurality of different fractions may be obtained, and each may be evaluated, by suitable bioassaying procedures, to determine the anti-diabetic activity thereof.

A fraction having anti-diabetic activity may preferably be further fractionated such as by column chromatography using silica gel as an adsorption medium and a 9:1 chloroform:methanol solvent, and the resultant sub-fractions bioassayed for their anti-diabetic activity. A sub-fraction displaying anti-diabetic activity may, if desired, be further fractionated and purified, conveniently using a column chromatographic procedure with silica gel as the adsorption medium and a 9:1 ethylacetate:hexane solvent. The resultant purified fractions may again be evaluated by suitable bioassay procedures for their anti-diabetic activity.

The inventors have found that at least one such purified fraction has superior anti-diabetic activity, and the active principle in the fraction was identified by conventional chemical techniques including nuclear magnetic resonance, and was found to be mangiferin. When this fraction is combined with a fraction comprising hesperidin a synergistic effect on diabetes is achieved, and further when the molar ration between mangiferin and hesperidin is from 1:1 to 2:1 the best effect is obtained.

The extract may be dried to remove moisture, e.g. by spray-drying, freeze-drying or vacuum drying, to form a free-flowing powder.

The invention and its efficacy is further described with the following examples.

### EXPERIMENT I

### Plant extract (laboratory scale)

Green plant material refers to plant material that is dried in such a manner to prevent enzymatic/chemical oxidation of the plant polyphenols and in particular the xanthones and flavonoids. Different drying procedures can be used.

Oxidised plant material refers to plant material that is oxidised for several hours after cutting of leaves and stems. The latter process initiates enzymatic and chemical oxidation of the polyphenols.. A water/enzyme (s) mixture can be added to aid enzymatic/chemical changes that takes place during the oxidation step of processing.

In the below examples the plant extract used have been obtained by the following procedure.

The preparation procedure for extract used involved extracting 10 kg of milled, green C. *intermedia* leaves and stems with 100 kg water at 95-98°C for 30 min (final extract temperature ca. 70°C after extraction) with continuous stirring, followed by continuous centrifugation to separate the extract and insoluble plant material. The extract was cooled to room temperature with a heat exchanger, where after aliquots were frozen and stored in PET bottles at -20°C until use. A small volume was also freeze-dried in a laboratory freeze-drier for in vitro testing.

### 1. Tissue Culture: in vitro assay models (cell lines) for anti diabetic screening

Diabetes is a multi factorial disease that affects many organs differently. Therefore, a combination of three cell lines, each representing a different organ affected by diabetes, plus a unique but simple, non-radioactive method, are used to measure glucose utilization, instead of glucose transport.

### Method

Our method measures the utilisation of glucose by 3T3-L1 adipocytes, Chang liver cells and C2C12 myocytes in 96-well plates over a period of one to three hours, depending on the cell line. This is done by starving the cells, adding glucose and then monitoring the disappearance of glucose from the incubation medium in the presence and absence of test samples. The adipocytes and liver cells are pre-exposed to the test samples for 48 hours, prior to the measurement of glucose utilization, to ensure that any chronic effects are also considered. Viability of cells exposed to the extracts for 48 hours is compared to that of control cells, allowing the identification of potentially toxic samples. Longer incubation times, and measurement of glucose utilisation or metabolism, allow detection of alterations in any of the pathways that are involved in glucose metabolism, not only in glucose transport. Table 1 summarises the responses measured in the three cell lines. This combination covers the mechanism of action of all classes of hypoglycaemic drugs currently available for the treatment of type 2 diabetes, except those that reduce intestinal glucose absorption.

**Table 1. Summary of the three cell lines used for routine anti diabetic screening**

| **CELL LINE** | **RESPONSE MEASURED** | **GLUCOSE TRANSPORTER** | **ACUTE/CHRONIC EFFECTS MEASURED** | **ACTION SIMILAR TO** |
|---|---|---|---|---|
| 3T3-L1 fat cell | Glucose utilisation | GLUT4 (insulin responsive) | Chronic | Thiazolidinedione s Insulin |
| Chang liver | Glucose utilisation | GLUT2 (not insulin responsive) | Chronic | Biguanides |
| C2C12 muscle | Glucose utilisation | GLUT4 | Acute | Insulin |

Extracts obtained were found to be effective in increasing glucose uptake in 3T3-L1 fat cells, displaying activity similar to Thiazolidenes and insulin. The extracts were also effective in the CHANG liver cells, displaying activity similar to Biguanides.

### 2. Streptozotocin model (T1D) or late stage T2D

Adult male Wistar rats (200 -250g) were used throughout the studies. Adult male Wistar rats were injected intra muscularly with streptozocin (STZ), at a dose of 36 mg /kg, to reduce or deplete their insulin producing cell numbers and induce hyperglycaemia at levels typical of type 1 diabetes or late stage T2D. Rats were fasted for 3 hours but were given drinking water ad libitum. After 72 hours of STZ injection, blood samples were taken from the tail vein. Plasma glucose concentrations were determined with a glucometer (Precision Q.I.D.) (Abbott Laboratories) using the glucose oxidase method. Rats with a blood glucose level of more than 300% of the fasting level were considered diabetic and were selected for the studies.

### Acute effect of the plant extract on plasma glucose in STZ rats

Diabetic rats were injected intraperitoneally with 20 mg/kg sodium pentobarbital to induce a lightly anaesthetized state. Approximately 10 to 15 minutes later, the rats were sufficiently calm to allow easy and stress-free handling, but with swallow-reflex intact. A Teflon gavage catheter was placed into the stomach, via the mouth and esophagus, and 1 ml of water containing the required extract was injected directly into the stomach. An additional volume of approximately 200 ul of water was then injected to flush any remaining extract from the gavage catheter. The catheter was then promptly removed and the rat placed in its cage for recovery. Group A was given normal saline, group B was given 5 mg/kg of the plant extract, group C was given 25 mg/kg of the plant extract and group D was given 50 mg/kg of the plant extract. Plasma glucose in mmol/l was measured at intervals of 1, 2, 3, 4, 5 and 6 hours. In adult male Wistar rats, injected with streptozotocin to reduce or deplete B-cells, an acute oral administration of the extract elicited a progressive reduction in plasma glucose over 6 hours..

### Oral Glucose Tolerance Test (OGTT)

Diabetic STZ rats, fasted for 16 hours received 25 mg/kg of the plant extract per gavage under light anesthesia (fluothane). After three hours the animals received an oral glucose bolus of 1 g/kg. Plasma glucose levels in mmol/l were determined at 0, 1, 5, 10, 15, 20, 30, 60 and 120 minutes. Results of the OGTT (1 g/kg glucose) performed three hours, after a single 25 mg/kg oral dose, showed that the plasma glucose levels of the STZ rats were lower when compared to control glucose values at all the time points taken (see Figure 13).

### 3. Obesity/insulin resistance rat model (T2D)

At weaning (three weeks old), male Wistar rats were fed an obesity inducing diet *ad libitum* for twelve weeks to induce symptoms typical of early stage T2D. After twelve weeks, blood was collected for baseline measurements.

### Administration of the extract

The extract was defrosted and diluted with deionised water to contain 0.54 (C1), 1.08 (C2), 1.79(C3), 2.15(C4) and 2.67% (C5) the extract, respectively.

### Administering of metformin

Metformin hydrochloride (850 mg, Rolab, Johannesburg) was dissolved in 85 ml of distilled water. Dissolved metformin was given with 30 ml of water each day. The dosage was calculated at 10 mg/ml in distilled water and rats were given 2 µl per gram body weight which is equivalent to 20 mg/kg body weight.

### Administering of rosiglitazone

Avandia rosiglitazone maleate (4 mg, GlaxoSmithKline, Bryanston, South Africa) was dissolved in 1 ml acid phosphate buffer pH 2.3. A dissolved tablet was given with 30 ml water in a dosage of 4 mg/kg body weight.

### Monitoring of extract, metformin, rosiglitazone and liquid intake

The amount of liquid consumed was monitored throughout the treatment period in all groups. At the same time every morning, liquid was measured before giving to animals. The liquid was measured every two days during the week and after three days during the weekend. The average intake was calculated as average of liquid taken in per week. Intake was recorded as the difference between the quantity given and the remaining volume.

### Fasting plasma glucose

Animals were fasted for three hours. The tip of the tail was cut using a pair of scissors and a drop of blood was used to determine the glucose level using a glucometer Precision Q.I.D (Abbott Laboratories).

### Blood collection

Animals were anaesthetised by inhalation of 98% oxygen and 2% fluothane (AstraZeneca Pharmaceuticals). The tail tip was amputated and blood collected into Epindorff tubes and stored on ice. Thereafter it was centrifuged at 2500 rpm for 15 minutes. Using a micropipette, 50 µl aliquots were placed in Nunc tubes and stored at minus 80°C until used.

### Serum for insulin measurements

After centrifugation at 4°C, plasma samples were stored at -20 °C until assayed for insulin which was measured by RIA using ¹²⁵I-labelled human insulin as tracer, and rat insulin as standard (Linco Research, St. Charles, MO. U.S.A.).

### Intravenous glucose tolerance test (IVGTT)

Animals were anaesthetised by inhalation of 98% oxygen and 2% fluothane (AstraZeneca Pharmaceuticals). The tip of the tail was amputated and baseline glucose was measured and recorded. Intravenous injection of 50% glucose at a dose of 0.5 mg/kg was performed over 20 seconds. Blood glucose measurements were taken at 1, 2, 3, 5, 10, 20, 30, 40, 50 and 60 minutes. Thereafter the rats were euthanased.

### Tissue processing

Pancreata, from five animals of each group, were collected after being treated with extract for 3 months. The whole pancreas was removed and fixed overnight in 4% buffered Formalin and embedded in paraffin. Serial 4 um thick sections were cut for immunocytochemistry and image analysis.

### Double Immunocytochemistry

Serial sections were de-waxed with xylene and hydrated through descending grades of ethanol. Sections on slides were transferred to 50 mM Tris-buffered-saline (TBS), pH 7.4, in a staining jar and were double immunostained for insulin (Sigma) and glucagon (Dako) using avidin D-biotinylated horseradish peroxidase (Vectastain) and streptavidin-biotin-complex/alkaline phosphatase (Dako). Beta cells were defined as insulin positive and alpha cells as glucacon positive cells. Beta cells were visualized with Fuchin red (red deposit) (Dako) and alpha cells with DAB (brown deposit) (Dako). Method controls involved omission, respectively, of primary antiserum, the second layer antibody, or the avidin D-biotinylated horseradish peroxidase complex/ streptavidin-biotin-complex/alkaline phosphatase.

### Image Analysis

Beta and alpha cells were measured on the same sections. Computer assisted measurements were taken with a standard Zeiss light microscope, that was linked to a video camera. The areas for insulin- and glucagon-positive cells were measured on the entire section. Beta and alpha cell volumes in the pancreases were calculated in each of the sections as the ratio of insulin- and alpha-positive area to the total pancreas area measured. Beta and alpha cell sizes were calculated by dividing the total area measured by the number of nuclei counted. The distribution of islet sizes was classified as follow: (< 2500 µm), (2500 µm <7500 µm), (7500 µm <12500µm), (12500µm <20000 µm) and (> 20000 µm). Islet density was determined by calculating total tissue area divided by the number of islets counted.

### Statistical Analysis

A two-tailed unpaired Student's t-test was used to test the significance of the results.

### Overall result

Fasting glucose levels were significantly reduced in Obesity/Insulin resistant rats treated with the extract of the present specification. In several cases, the reduction was greater with the extract than with Metformin or Rosiglitazone.

### CONCLUSIONS

Similar efficacy to Thiazolidines and Insulin (in fat cell studies) and Biguanides (in Chang liver cells) in increased glucose uptake has been demonstrated with the plant extract.

Treatment with the extract was, in some cases, more effective than Metformin and Rosiglitazone in reducing plasma glucose levels in streptozotocin treated (late stage T2D) and insulin resistant rats.

The extract displays encouraging efficacy in normalizing compromised glucose levels.

### EXPERIMENT II

The present experiment elucidates the optimal dose of the plant extract for reducing plasma glucose levels. In this experiment monkeys were used in order to resemble the situation of a human as closely as possible.

### Justification and validation for use

The vervet monkey (*Chlorocebus aethiops*), also called African Green monkey, is one of two African nonhuman primate species, most commonly utilized in biomedical research and endemic to Southern Africa. With some exceptions, the use of nonhuman primates internationally has been influenced more by geopolitical and logistical rather than biological considerations. The vervet monkey is taxonomically closely related to the macaques (i.e. rhesus), since all belong to the same subfamily (Fairbanks 2002). Although vervet monkeys are used in many fields including virology, bacteriology, parasitology, neurology, toxicology, reproduction and cell biology, they have proven to be particularly useful in the areas of cardiovascular and metabolic disease. As a result, the literature abounds with information and data, validating this well established model (de Vries *et al.* 2007, Fairbanks 2002, Fincham *et al.* 1998, Kavanagh et al. 2007, Louw *et al.* 1997, Martin *et al.* 1990, Rudel *et al.* 1981, Smuts *et al.* 1992, Suckling and Jackson 1993, Wallace *et al.* 2005, Weight *et al.* 1988).

It is important to stress that vervet monkeys develop spontaneous obesity, insulin resistance and type 2 diabetes (Fairbanks 2002, Francis *et al.* 1999, Kavannagh *et al.* 2007, Tigno *et al.* 2005). As much as 25% females and 16% males of a particular colony have been reported to be obese (Kavannagh *et al.* 2007). As humans, nonhuman primates develop all the associated complications including renal, vascular and neurological (Tigno *et al.* 2005). In some vervet monkey populations as much as 4% can have abnormally high plasma glucose concentrations (Fairbanks 2002, Kavanagh *et al.* 2007), and there is a strong positive association between waist circumference, increased plasma insulin as well as plasma triglyceride concentrations (Kavannagh *et al.* 2007). It has also been established that obesity and associated plasma lipids and other risk factors are heritable in this species (Kavannagh *et al.* 2007).

It is also important to note that vervet monkeys develop spontanous atherosclerosis and respond well to experimental nutritional manipulations to produce dyslipidaemia and ultimately atherosclerosis (Fairbanks 2002, Rudel *et al.* 1981, Fincham *et al.* 1998, Suckling and Jackson 1993). The associated underlying mechanisms and lesions model the human condition (Fincham *et al.* 1996, Fincham *et al.* 1998, Rudel *et al.* 1981), and vervet monkeys are responsive to well recorded older and more novel pharmacological intervention strategies (Fincham *et al.* 1996, St. Clair *et al.* 1981, Wallace *et al.* 2005).

### Primate Management

In the present study primate management and care was according to the documented Standard Operating Procedures (Mdhluli 2005) and the MRC Guidelines on the Use of Animals in Research and Training, the National Code for Animal Use in Research, Education, Diagnosis and Testing of Drugs and related Substances in South Africa, and the Veterinary and Para-Veterinary Professions Act of 1997: Rules relating to the practicing of the Para-Veterinary Profession of Laboratory Animal Technologist.

### Choice of monkeys and their permanent identification

All individuals selected for this study were healthy adult males and females, 2nd generation captive bred, with an average weight of 5.56 kg (± 0.724) and 3.16 kg (± 0.266), respectively. Average age was 12 years for males and 7 years for females. Female vervet monkeys mature sexually in captivity at about 2.5 to 3.0 years of age, and males at about 3.0 to 4.0 years (Eley 1992).

All individuals were free of overt pathology as judged by physical examination and previous clinical record, of normal weight for age, and identified with a permanent number in ink tattoo. Additionally, cages were marked according to individual number, group designation, and experiment number.

### Environmental conditions

All vervet monkeys used in this study were maintained in the Primate Unit of the Technology and Innovation directorate of the MRC under identical housing conditions. The facility consists of 14 fully air conditioned animal rooms in a closed indoor environment that is maintained at 24-26°C, a humidity of about 45%, about 15-20 air changes per hour and a photoperiod of 12 hours. All rooms are kept under positive pressure and have separate air supply.

### Housing

Caging was singly for the duration of the study, and consisted of 90 x 70 x 120cm suspended galvanized steel cages, with 24 monkeys being maintained in one room. Animal rooms were sanitized once daily. The cage size is consistent with the requirements of the South African National Code for single animals.

### Food and water

Water was available *ad lib* via an automatic watering device. The maize meal based maintenance diet was produced in the Primate Unit, and has supported good growth and reproduction for three generations (Seier 1986). Seventy gram of maintenance diet consisting of dry maize meal, containing added micro- and macronutrients, was mixed with water to a stiff consistency and fed to the monkeys at 7:00 am, 11:00 am and another 70 g at 3:00 pm but without the added nutrients. This supplies 2412 kJ per monkey per day with 12% energy from protein, 20% from fat and 68% from carbohydrates. In addition, apples or oranges are fed at noon at 70 g/monkey/day. The detailed composition of the diet has been described previously (Fincham *et al.* 1987, Venter *et al.* 1993).

### Supporting facilities

All activities of the Primate Unit are fully physically separated by dedicated areas and rooms for cage sanitation, food preparation and storage, storage and formulation of compounds under investigation, procedures (i.e. blood sampling), operations (theatre) and necropsy.

### Environmental enrichment

Single cages are fitted with resting perches, 80 cm above the cage floor, foraging pans and communication panels, which enable grooming and physical contact with conspecifics. Exercise cages measuring 90 x 70 x 200 cm area available three times/week to each monkey and enable leaving the home cages and engaging in certain activities that are not possible in home cage (Seier and de Lange 1996). The cage also enables 360° communication with all other animals in the room as well as the adjacent room (through glass panels). Soft music and bird sounds are broadcasted into each animal room to relieve auditory monotony. Other enrichment methods have been described previously (Seier *et al.* 2004).

### Health and disease control

All animals were tested for TB four times per year by injecting 3000 units of PPD intradermally into the upper eyelid. MRC staff and students, as well as service providers, were tested for TB twice per year by culturing bronchial secretions for Mycobacteria. According to Primate Unit SOPs, additional bacteriological and serological testing is carried out on the vervet monkeys from time to time, and is consistent with accepted standards (FELASA 1998). This includes testing for Shigella, Salmonella, Campylobacter and Yersinia.

### Handling of vervet monkeys, administration of substance, and collection of samples

Procedures and handling of the vervet monkeys were according to Primate Unit SOPs, and all other guidelines mentioned in the preamble. They are carried out and/or supervised by fully qualified and experienced laboratory animal technologists who are registered with the SA Veterinary Council in laboratory animal technology.

### Observations

All animals have been observed three times/day to determine potential physical side effects of the treatment. The following criteria were used: posture, coordination, locomotion, activity, behaviour (alert, fearful, aggressive, confused, depressed, vocalization), discharge from orifices, appetite, condition of feces and urine.

### PREPARATIONS OF GMP ARC137

Preparation of a plant extract was made for testing of bio-activity in the primate model. Organic certified green *Cyclopia subternata* leaves and stems, pretested for the bio-activity, was processed in a cGMP facility.

### Manufacturing details

The manufacturing process for comprised the following unit operations: extraction of the plant material, separation of the extract and small particulate matter, evaporation, HTST sterilization of the concentrate, vacuum drying of the concentrate and sieving of the final product powder.

### Preparation of extract

The plant material was extracted in two subsets of 200 kg per percolator. Purified water (2000 kg) preheated to 93°C, was introduced into the percolator from the top at a rate of 1:10 and the resulting extract was circulated for 35 min. At completion of extraction sub set 1 was drained and an additional 200 kg of purified water flushed through to give a total of 1889 kg of aqueous extract with a dry residue of 3.0% (dry extract yield - 28.6%). Sub set 2 was extracted in a similar manner, giving 1889 kg aqueous extract with a dry residue of 2.92% (dry extract yield - 27.6%).

The extract was centrifuged warm at a flow rate of 1000 l/h, with a draining cycle of the sediment every 30 min. The final extract recovered after centrifugation was 3753 kg with a dry residue yield of 2.98%.

After centrifugation the aqueous extract at an inlet temperature of ca. 78°C was concentrated with a plate evaporator under vacuum at <55°C to 472 kg and a dry residue of 23.98%.

The concentrate was HTST sterilized at 121-123°C (ca 68 s) at a flow rate of 385-445 l/h. The concentrate was cooled to <25°C after sterilization. Purified water was used to wash out the plant, giving a final sterilized concentrate of 485 kg with a dry residue of 22.93%.

The sterilized concentrate was dried in a vacuum drier at a product temperature <46°C for 24 h. After drying the power was sieved through two sieves (2 mm followed by 0.5 mm) to remove lumps that formed during the rotation of the paddles in the vacuum drier. The sieved powder was placed in two PE bags (34.45 kg; 46.35 kg), which were then sealed separately in aluminium coated fibre drums.

### Plant material

The HPLC fingerprint of the plant material was determined on an aqueous extract. The extract was prepared by extracting the plant material with deionised, purified water at 90-93°C (giving 1:10 ratio) for 30 min in a water bath, filtering warm through Whatman no. 4 filter paper and frozen, whereafter it was freeze-dried. For HPLC analysis the freeze-dried extract was reconstituted in purified water.

The HPLC fingerprint of the final product (GMP ARC137) was determined on the extract reconstituted in purified water.

### RESULTS

Figure 1 depicts the HPLC fingerprint of the laboratory-scale aqueous extract of the plant material samples at the relevant detection wavelengths, 288 and 350 nm. Figure 2 depicts the HPLC fingerprint of the final GMP dry extract, at the same relevant detection wavelengths.

### VERVET MONKEY DOSE OPTIMISATION STUDY

### Controls

Three monkeys where randomised into a control group. The monkeys in the control group received a 70 g ration of maintenance diet (molded into ball) but without the dry extract, three times a day for 7 days. On day 7 after the monkeys were fasted overnight (13 hours), a baseline blood sample was collected before the monkeys were fed the ball maintenance diet bolus. Thereafter blood samples were collected hourly for a six hour period by femoral venipuncture under Ketamine anaesthesia (10 mg/kg bodyweight intramuscular injection).

### Discussion of Control Group Results

Monkey #88 showed a fasting baseline blood glucose value of 4.5 mMol/l that was stable for the first two hours of the monitoring period before the blood glucose levels showed a marked decline by the third hour falling to 2.9 mMol/l, a 35.56% decline of blood glucose percentage, before increasing slightly to 3.6 after 4 hours and 3.9 mMol/l after five and six hours. Monkey #1082, with a slightly elevated fasting baseline blood glucose value of 5.2 mMol/l showed a further slight increase in blood glucose values for the first two hours increasing to 5.3 mMol/l and 5.6 mMol/l at one and two hours, respectively, before decreasing to 4.4 mMol/l at three hours. Blood glucose values continued to decline to 4.1 mMol/l and 3.5 mMol/l at four and five hours, respectively, before increasing slightly to 4.7 mMol/l at six hours. The highest reduction of 32.69% in the blood glucose percentage was seen at five hours. Monkey # 333 showed increased blood glucose levels above the baseline blood glucose level of 4.6 mMol/l at each hourly time point of the six hour monitoring period. At six hours the monkey became hyperglycaemic with a blood glucose level of 7.5 mMol/l, a 44.23% increase in blood glucose percentage calculated from the baseline blood glucose value. The increase in blood glucose is caused by gluconeogenisis which is a typical response of a diabetic animal to an extended period of fasting.

### Experimental groups

Nine monkeys randomized into four experimental groups receiving 1 mg/kg, 2.5 mg/kg, 5 mg/kg and 25 mg/kg GMP ARC137, three times a day. All the monkeys in the respective experimental groups received a pre-weighed aliquot of GMP ARC137 moulded into a 70 g ball of maintenance diet as a bolus, three times a day for 7 days. On day 7 after the monkeys were fasted overnight (13 hours), a baseline blood sample was collected before the monkeys were fed the maintenance diet bolus containing the respective amounts of dry extract. Thereafter blood samples were collected hourly for a six hour period by femoral venipuncture under Ketamine anaesthesia (10 mg/kg bodyweight intramuscular injection).

### Discussion of GMP ARC137 1.0 mg/kg Experimental Group Results

Monkey #1084 showed an elevated fasting baseline blood glucose value of 5.7 mMol/l. At two hours, after an initial marginal decline of the one hour blood glucose level to 5.3 mMol/l, the blood glucose levels decreased sharply to 2.8mMol/l, a 50.88 % decline in the blood glucose percentage, before recovering to 3.9 mMol/l at 3 hours after receiving the bolus containing 1.0 mg/kg GMP ARC137. Thereafter the blood glucose levels stabilized at 4.2 and 4.5 mMol/l at four and five hours, respectively, before increasing to 5.3 mMol/l at 6 hours. Monkey #1081 showed a hyperglycaemic fasting baseline blood glucose level of 7.5 mMol/l. One hour after receiving the bolus containing 1.0 mg/kg GMP ARC137 the blood glucose level decreased by 1.4 mMol/l, a 40% decline in the blood glucose percentage, and continued to decline by a further 1.6 mMol/l at two hours to 4.5 mMol/l. Blood glucose levels of 4.6 mMol/l was maintained for the next two hours before increasing slightly to 5.2 mMol/l at five hours. At six hours the blood glucose level decreased to 4.4 mMol/l, 41.33% (3.1 mMol/l) lower than the baseline value. Monkey #266 showed a moderately elevated baseline blood glucose of 5.3 mMol/l. At one and two hours after receiving the bolus containing 1.0 mg/kg GMP ARC137 the blood glucose values remained at 5.0 and 5.1 mMol/l, respectively before increasing to 5.7 mMol/l and 5.8 mMol/l at three and four hours respectively. At five hours the blood glucose level increased sharply to 7.3 mMol/l followed by a decrease to 5.5 mMol/l at six hours, still marginally higher than the baseline value of 5.3 mMol/l. In sharp contrast to the other two monkeys monkey #266 did not respond to GMP ARC137 at the given dose of 1 mg/kg. After very small initial reductions in the blood glucose percentage at one and two hours the glucose levels increased to a peak of 40.38% at five hours before declining to 5.77% at six hours.

### Discussion of 2.5 mg/kg GMP ARC137 Experimental Group Results

Monkey #78, with a fasting baseline blood glucose value of 4.6 mMol/l showed a 1 mMol/l (21.74%) decline of the blood glucose value one hour after receiving the bolus containing 2.5 mg/kg GMP 137 extract. These lower than baseline levels ranging between a 15.22% and 28.25% reduction in the blood glucose as calculated against the baseline values were maintained for the remainder of the six hour monitoring period. Monkey #1083 showed an increase in the blood glucose value to 5.0 mMol/l compared to the baseline blood glucose values of 4.4 mMol/l at one hour after receiving the bolus containing 2.5 mg/kg GMP ARC137. At two hours the blood glucose levels declined markedly by 1.8 mMol/l to 3.2 mMol/l and then declined further to 2.8 mMol/l by three hours. Thereafter blood glucose levels remained at around 3.0 mMol/l for the remaining monitoring period. Monkey #1084 showed an elevated fasting blood glucose baseline of 5.7 mMol/l. At one hour blood glucose level of 5.3 mMol/l remained elevated, however, at two hours after receiving the bolus containing 2.5 mg/kg GMP extract the blood glucose level decreased substantially to 3.2 mMol/l, a 43.6% reduction in blood glucose percentage. The blood glucose level then remained stable at 3.7 mMol/l, a level 35.7% lower than the baseline blood glucose value.

### Discussion of 5.0 mg/kg GMP ARC137 Experimental Group Results

Monkey #1079, after receiving the bolus containing 5 mg/kg GMP ARC137, showed an increase in blood glucose levels from a baseline value of 3.7mMol/l to 4.5 mMol/l and 4.6 mMol/l at one and two hours, respectively. At three hours the blood glucose level decreased to 3.3 mMol/l before increasing to 4.3 mMol/l at four hours and then stabilizing at near baseline values of 3.6 mMol/l and 3.8 mMol/l at five and six hours, respectively. Monkey #1081 showed a hyperglycaemic fasting baseline blood glucose level of 7.5mMol/l. One hour after receiving the bolus containing 5.0 mg/kg GMP ARC137 the blood glucose level was still high at 7.2 mMol/l. After 2 hours the blood glucose decreased sharply to 4.6 mMol/l before stabilizing between 5 mMol/l and 6 mMol/l for the last 4 hours of the monitoring period.

### Discussion of 25 mg/kg GMP ARC137 Experimental Group Results

Monkey #119 showed a moderately elevated fasting baseline blood glucose value of 5.5 mMol/l. One hour after receiving the bolus containing 25 mg/kg GMP ARC137 the blood glucose level had decreased to 4.8 mMol/l. A blood glucose level of 4.9 mMol/l was maintained for a further two hours before increasing slightly to 5.4 mMol/l at four hours. The blood glucose levels then decreased slightly to 4.3 mMol/l at 5 hours before increasing to 4.9 mMol/l at six hours. Monkey #258 showed an extreme hyperglycaemic blood glucose baseline value of 21.2 mMol/l, which is clinically consistent with a late type 2 or type 1 diabetic requiring insulin support. One hour after receiving the bolus containing 25 mg/kg GMP ARC137 the blood glucose levels decreased by 4 mMol/l to 17.2 mMol/l, then stabilized at 18.2 mMol/l at two hours. At three hours the blood glucose dropped by a further 4 mMol/l to 14.2 mMol/l, representing a 33.2% decline in blood glucose percentage calculated against the baseline blood glucose values. At four hours the blood glucose values increased slightly to 16.8 mMol/l, which is still 20.75% lower than the baseline value before decreasing further to 14.3 and 13.3 mMol/l at five and six hours, respectively. Monkey #266 showed a moderately elevated fasting baseline blood glucose level of 5.2 mMol/l. This value increased further to 5.8 mMol/l one hour after receiving the bolus containing 25 mg/kg GMP ARC137. However, the blood glucose level dropped to 4.2 mMol/l and 4.5 mMol/l at two and three hours respectively. Thereafter levels were not maintained for the rest of the monitoring period, increasing to 6.0, 7.0 and 6.1 mMol/l at four, five and six hours respectively. The increased values at hours five and six are strongly suggestive of gluconeogenesis induced in a diabetic animal by an extended period of fasting.

### SUMMARY OF RESULTS

### Control Group

In the control group none of the three monkeys tested showed a decline from the baseline values during the first two hours after receiving the portion of moulded maintenance diet. Thereafter, in two of the three monkeys, a reduction in the glucose values compared to their baseline values, was observed.

### Blood glucose values in the 1.0 mg/kg GMP ARC137 treatment group.

Results of the 1 mg/kg GMP ARC137 group showed a marked reduction in the blood glucose values compared to the baseline blood glucose levels in two of the monkeys, over the six hour monitoring period. Monkey #266 failed to respond to the GMP ARC137 treatment at 1 mg/kg .

### Blood glucose values in the 2.5 mg/kg GMP ARC137 treatment group.

The blood glucose levels of the three monkeys receiving 2.5 mg/kg GMP ARC137 showed a marked reduction in their blood glucose levels over the first two hours and thereafter the lower blood glucose levels were maintained for the remaining monitoring period.

### Blood glucose values in the 5 mg/kg GMP ARC137 treatment group.

Monkey #263 of the 5 mg/kg GMP ARC137 treatment group, after initially eating the treatment bolus, refused the treatment the morning of the blood glucose determinations and had to be excluded from the study. Monkey #1081 with high fasting baseline glucose (hyperglycaemic) showed a marked reduction of blood glucose levels two hours after receiving 5 mg/kg GMP ARC137. These levels were maintained for the duration of the monitoring period. Monkey #1079 had normoglycaemic starting blood glucose levels which were maintained over the monitoring period.

### Blood glucose values in the 25 mg/kg GMP ARC137 treatment group.

Monkey #258 showed severed hyperglycaemia with a fasting baseline value of 21.2 mMol/l. Althought the blood glucose levels decline at each time point monitored, the blood glucose level at six hours was still at 13.3 mMol/l. This monkey was excluded from the study. The remaining two monkeys failed to respond to the treatment at the given dose.

### Comparison of results in monkeys receiving different dosages of the GMP ARC137.

Monkey #266 failed to respond to the GMP ARC137 treatment at 1 mg/kg and at 25 mg/kg. Monkey #1081, receiving 1mg/kg and 5mg/kg GMP ARC137, showed marked decreased blood glucose levels over the six hours monitored. In comparison, the GMP ARC137 treatment at 1 mg/kg showed the greatest reduction and maintained the lowest blood glucose levels for the six hour monitoring period.

Figure 15 shows % reduction in plasma glucose over a 6 hour period following treatment with different dosages of the extract (ARC137) for 7 days in the Vervet monkey (average data).

### Conclusion

In the small pilot study, GMP ARC137 in the dose range of 1 - 2.5 mg/kg was the most effective in reducing the blood glucose of a nonhuman primate (*Chlorocebus aethiops*)*.*

## Claims

1. A pharmaceutical preparation in dosage unit form for oral administration for use in the treatment of diabetes, comprising, per dosage unit, a therapeutically effective amount of plant extract, said plant extract obtainable by a method comprising the steps of:
(a) providing *Cyclopia* plants or portions thereof,
(b) combining said plants or portions thereof with a nontoxic solvent appropriate for solubilizing said plant extract, and heating the solvent to a temperature between 60 °C and 95 °C;
(c) recovering said plant extract, and
(d) drying;
wherein said therapeutically effective amount is from 0.1 milligram to 25 milligrams per kilogram body weight.

2. The preparation of claim 1, wherein said therapeutically effective amount is from about 1 milligram to about 5 milligrams per kilogram body weight.

3. The preparation of claim 1, wherein said therapeutically effective amount is from about 1 milligram to about 2.5 milligrams per kilogram body weight.

4. Plant extract for use in the treatment of diabetes, said extract obtainable by a method comprising the steps of:
(a) providing *Cyclopia* plants or portions thereof,
(b) combining said plants or portions thereof with a nontoxic solvent appropriate for solubilizing said plant extract, and heating the solvent to a temperature between 60 °C and 95 °C;
(c) recovering said plant extract, and
(d) optionally drying;
wherein said extract is to be orally administered in an from 0.1 milligram to about 25 milligrams per kilogram body weight.

5. Plant extract according to claim 4, wherein said extract is to be administered in an amount from about 1 milligram to about 5 milligrams per kilogram body weight.

6. Plant extract according to claim 4 or 5, wherein said extract is to be administered in an amount from about 1 milligram to about 2.5 milligrams per kilogram body weight.

## Patentansprüche

1. Pharmazeutische Zubereitung in Form von Dosierungseinheiten für die orale Verabreichung zur Verwendung bei der Behandlung von Diabetes, umfassend, pro Dosierungseinheit, eine therapeutisch wirksame Menge an Pflanzenextrakt, wobei der Pflanzenextrakt durch eine Methode erhältlich ist, umfassend die Schritte:
(a) des Bereitstellens von *Cyclopia-*Pflanzen oder Teilen davon;
(b) des Kombinierens der Pflanzen oder Teile davon mit einem nicht-toxischen Lösungsmittel geeignet zur Solubilisierung des Pflanzenextrakts, und des Erhitzens des Lösungsmittels auf eine Temperatur zwischen 60°C und 95°C;
(c) des Rückgewinnens des Pflanzenextrakts; und
(d) des Trocknens;
wobei die therapeutisch wirksame Menge von 0,1 Milligramm bis zu 25 Milligramm pro Kilogramm Körpergewicht beträgt.

2. Zubereitung nach Anspruch 1, wobei die therapeutisch wirksame Menge von etwa 1 Milligramm bis zu etwa 5 Milligramm pro Kilogramm Körpergewicht beträgt.

3. Zubereitung nach Anspruch 1, wobei die therapeutisch wirksame Menge von etwa 1 Milligramm bis zu etwa 2,5 Milligramm pro Kilogramm Körpergewicht beträgt.

4. Pflanzenextrakt zur Verwendung bei der Behandlung von Diabetes, wobei das Extrakt durch eine Methode erhältlich ist, umfassend die Schritte:
(a) des Bereitstellens von *Cyclopia*-Pflanzen oder Teilen davon;
(b) des Kombinierens der Pflanzen oder Teile davon mit einem nicht-toxischen Lösungsmittel geeignet zur Solubilisierung des Pflanzenextrakts, und des Erhitzens des Lösungsmittels auf eine Temperatur zwischen 60°C und 95°C;
(c) des Rückgewinnens des Pflanzenextrakts; und
(d) optional: des Trocknens;
wobei der Extrakt oral in einer Menge von etwa 0,1 Milligramm bis zu etwa 25 Milligramm pro Kilogramm Körpergewicht verabreicht werden soll.

5. Pflanzenextrakt nach Anspruch 4, wobei der Extrakt in einer Menge von etwa 1 Milligramm bis zu etwa 5 Milligramm pro Kilogramm Körpergewicht verabreicht werden soll.

6. Pflanzenextrakt nach Anspruch 4 oder 5, wobei der Extrakt in einer Menge von etwa 1 Milligramm bis zu etwa 2,5 Milligramm pro Kilogramm Körpergewicht verabreicht werden soll.

## Revendications

1. Une préparation pharmaceutique en forme d'unité de dosage pour l'administration orale destinée à être utilisée dans le traitement du diabète, comprenant, par l'unité de dosage, une quantité thérapeutiquement efficace, du point de vue thérapeutique, d'un extrait de plante, ledit extrait de plante susceptible d'être obtenu par un procédé comprenant les étapes :
(a) la provision de plantes *Cyclopia* ou de parties de celles-ci;
(b) la combinaison des plantes ou des parties de celles-ci avec un solvant non-toxique convenant à solubiliser l'extrait de plantes, et le chauffage du solvant à une température entre 60°C et 95°C ;
(c) la récupération de l'extrait de plantes; et
(d) le séchage;
dans laquelle la quantité thérapeutiquement efficace est de 0,1 milligramme à 25 milligramme par kilogramme de poids corporel.

2. Préparation selon la revendication 1, dans laquelle la quantité thérapeutiquement efficace est d'environ 1 milligramme à environ 5 milligramme par kilogramme de poids corporel.

3. Préparation selon la revendication 1, dans laquelle la quantité thérapeutiquement efficace est d'environ 1 milligramme à environ 2,5 milligramme par kilogramme de poids corporel.

4. Un extrait de plantes destiné à être utilisé dans le traitement du diabète, ledit extrait de plantes susceptible d'être obtenu par un procédé comprenant les étapes :
(a) la provision de plantes *Cyclopia* ou de parties de celles-ci;
(b) la combinaison des plantes ou des parties de celles-ci avec un solvant non-toxique convenant à solubiliser l'extrait de plantes, et le chauffage du solvant à une température entre 60°C et 95°C ;
(c) la récupération de l'extrait de plantes; et
(d) optionellement le séchage;
dans lequel dit extrait est à administrer oralement dans une quantité de 0,1 milligramme à 25 milligramme par kilogramme de poids corporel.

5. L'extrait de plantes selon la revendication 4, dans lequel dit extrait est à administrer dans une quantité d'environ 1 milligramme à 5 milligramme par kilogramme de poids corporel.

6. L'extrait de plantes selon la revendication 4 ou 5, dans lequel dit extrait est à administrer dans une quantité d'environ 1 milligramme à 2,5 milligramme par kilogramme de poids corporel.
